# EUROPEAN PATENT APPLICATION

(11) **EP 4 501 929 A1**
(43) Date of publication of application: **05.02.2025**
(21) Application number: 23778296.6
(22) Date of filing: 29.03.2023
(51) Int. Cl.: C07D 417/12, A61K 31/427, A61K 31/506, A61P 13/00, A61P 25/04, A61P 29/00

(54) **P2X3 INHIBITOR COMPOUND, SALT THEREOF, POLYMORPH THEREOF AND USE THEREOF**

(30) Priority: 29.03.2022 CN 202210326113; 21.03.2023 CN 202310281376
(71) Applicant: Humanwell Healthcare (Group) Co., Ltd., Wuhan, Hubei 430075 (CN); Wuhan Humanwell Innovative Drug Research and Development Center Limited Company, Wuhan, Hubei 430075 (CN)
(72) Inventor: ZHANG, Xuejun, Wuhan, Hubei 430075 (CN); ZANG, Yang, Wuhan, Hubei 430075 (CN); LI, Qun, Wuhan, Hubei 430075 (CN); LEI, Sijun, Wuhan, Hubei 430075 (CN); LIU, Lifei, Wuhan, Hubei 430075 (CN); LI, Yuan, Wuhan, Hubei 430075 (CN); XIA, Qingfeng, Wuhan, Hubei 430075 (CN); LI, Li'e, Wuhan, Hubei 430075 (CN); YANG, Jun, Wuhan, Hubei 430075 (CN)
(74) Representative: Gille Hrabal Partnerschaftsgesellschaft mbB Patentanwälte
(86) International application number: PCT/CN2023/084726
(87) International publication number: WO 2023/185931

(57) **Abstract**

The present invention relates to a P2X3 inhibitor compound, a salt thereof, a polymorph thereof and use thereof. The present invention provides a crystalline form of a compound of formula I, which has good medicinal properties. A pharmaceutically acceptable salt of the compound of formula I is also obtained, and a crystalline form product of the salt is further obtained, such as a hydrochloride crystalline form A, a maleate crystalline form A, a p-toluenesulfonate crystalline form A, a benzene sulfonate crystalline form A, or a malonate crystalline form A. The present invention has great significance for developing effective therapeutic drugs.

## Description

The present application claims priority to the following prior applications: the prior application with the patent application No. 202210326113.5 and entitled "P2X3 INHIBITOR COMPOUND, SALT THEREOF, POLYMORPH THEREOF AND USE THEREOF" filed with China National Intellectual Property Administration on March 29, 2022; and the prior application with the patent application No. 202310281376.3 and entitled "P2X3 INHIBITOR COMPOUND, SALT THEREOF, POLYMORPH THEREOF AND USE THEREOF" filed with China National Intellectual Property Administration on March 21, 2023, which are incorporated herein by reference in their entireties.

### TECHNICAL FIELD

The present disclosure belongs to the field of pharmaceuticals, and relates to a P2X3 inhibitor compound, a salt thereof, and a polymorph thereof, as well as a preparation method therefor and use thereof.

### BACKGROUND

P2X receptor is a non-selective ATP-gated ion channel receptor, i.e., a purinergic receptor, which can bind to extracellular ATPs mainly derived from damaged or inflamed tissues. This receptor is widely expressed in the nervous, immune, cardiovascular, skeletal, gastrointestinal, respiratory and endocrine systems and other systems, and is involved in a variety of physiological processes such as the regulation of heart rhythm and contractility, the regulation of vascular tone, the regulation of nociception (especially chronic pain), the contraction of vas deferens during ejaculation, the contraction of bladder during urination, the aggregation of platelets, the activation of macrophages, apoptosis, neuron-glial interactions, and the like. The P2X receptor described above includes: seven homologous receptors, i.e., P2X1, P2X2, P2X3, P2X4, P2X5, P2X6, and P2X7; and three heterologous receptors, i.e., P2X2/3, P2X4/6, and P2X1/5.

P2X3 is a subtype of the P2X receptor family, and is selectively expressed in dorsal root ganglia, spinal cord, and brain neurons of nerve endings, i.e., in primary sensory neurons with small to medium diameters.

Numerous studies have shown that activation of P2X3 and P2X2/3 expressed in the primary sensory neurons plays an important role in acute injury, hyperalgesia, and hypersensitivity in rodents. Many studies have shown that upregulation of P2X3 receptor expression may lead to the development of hyperalgesia and is involved in pain signaling. P2X3-knockout mice exhibit reduced pain responses, and in models of pain and inflammatory pain, P2X3 receptor antagonists demonstrate an alleviating effect on nociception.

P2X3 is distributed in primary afferent nerves around the airways and is capable of regulating cough. Studies have shown that ATPs released from damaged or inflamed airway tissues act on P2X3 receptors of primary neurons, triggering depolarization and action potentials. The propagation of these potentials causes the impulse to cough and thus induces coughing. P2X3 receptors play an important role in the hypersensitivity of the cough reflex. By antagonizing the binding to P2X3 receptors, the hypersensitivity of the cough reflex can be inhibited, thereby suppressing excessive coughing in patients with chronic cough. In addition, studies have also shown that P2X3 antagonists can treat chronic obstructive pulmonary disease, pulmonary fibrosis, pulmonary arterial hypertension, or asthma. Therefore, P2X3 antagonists are also promising to become new drugs for the treatment of the above-mentioned diseases.

It has been reported that P2X3 is involved in the afferent pathway that controls the bladder capacity reflex, and P2X3-knockout mice exhibit a significant decrease in urination frequency and a significant increase in bladder capacity. Therefore, the binding of P2X3 receptor-inhibiting antagonists to P2X3 receptors has effects in the treatment of urinary storage disorders and voiding disorders, such as overactive bladder. Therefore, P2X3 antagonists may be potential drugs for the treatment of overactive bladder and other related diseases.

P2X3 antagonists show great promise. Currently, commonly used clinical cough drugs gabapentin, morphine and amitriptyline or treatment with speech pathology can alleviate cough in many patients, but these therapies are not suitable for all patients. In addition, centrally acting drugs such as gabapentin and the like may cause adverse side effects and thus are not suitable for long-term medication. There is an urgent clinical need to develop chronic refractory cough drugs suitable for long-term medication, so as to provide doctors with more medication options. Therefore, the development of P2X3 antagonists is of great clinical significance.

Chinese patent application No. CN202111165441.3 discloses a compound of formula I with the following structure:

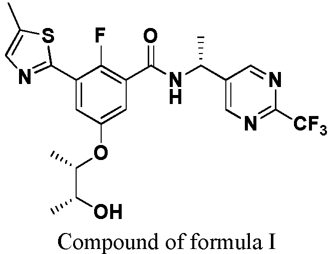

The compound of formula I is capable of effectively antagonizing the activity of a P2X3 receptor, and has wide application prospects for the manufacturing of a medicament for the treatment of P2X3-related diseases, so that further research on the compound of formula I as well as salt forms and crystalline forms thereof is of great significance for the development of effective therapeutic medicaments.

### SUMMARY

In order to address the problems in the prior art, in one aspect, the present disclosure provides a crystalline form of a compound of formula I or a pharmaceutically acceptable salt thereof, wherein the compound of formula I has a structure shown as follows:

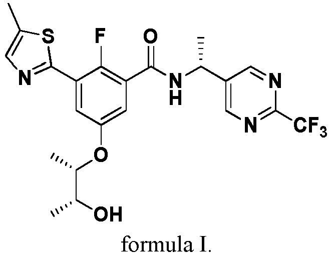

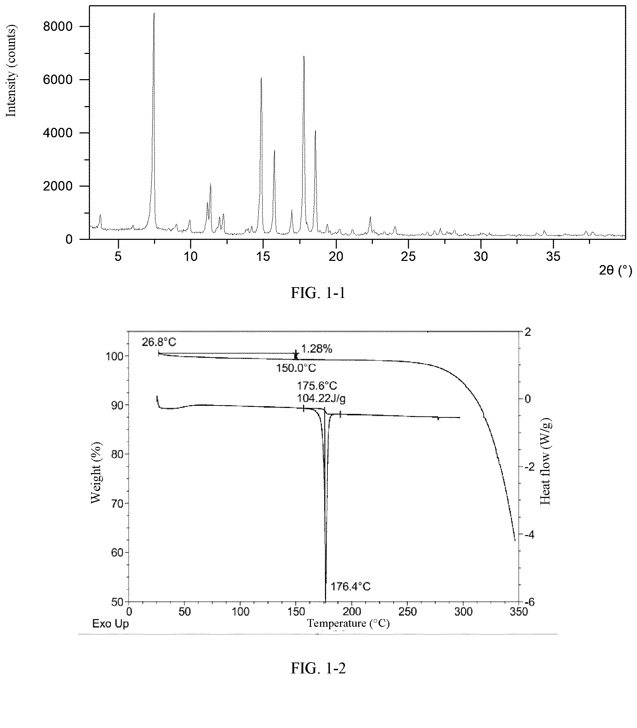

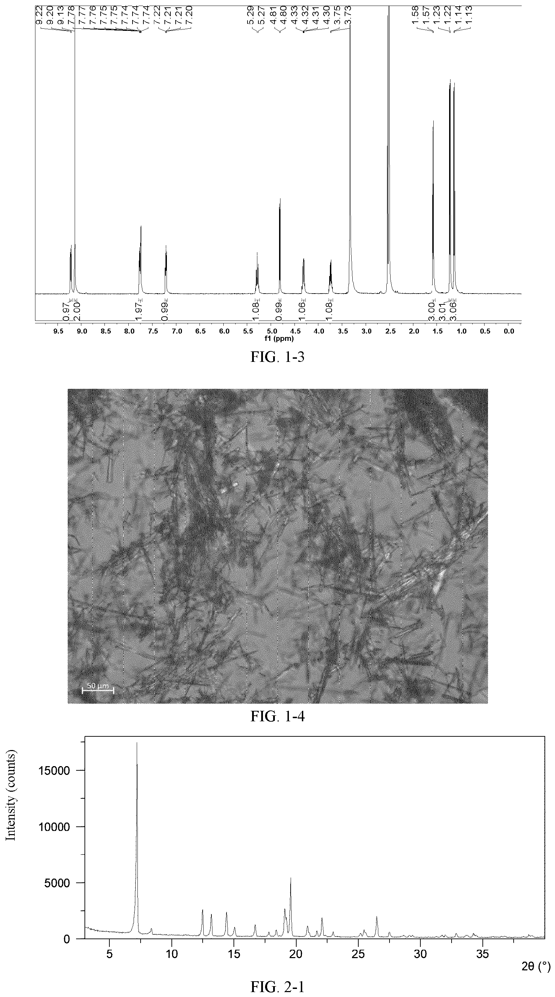

In some embodiments, the present disclosure provides free base crystalline form A of the compound of formula I, wherein the free base crystalline form A has an X-ray powder diffraction pattern comprising diffraction peaks at 2Θ±0.2° diffraction angles of 7.44°, 14.87°, 15.77°, 17.81°, and 18.61°; further, the free base crystalline form A has an X-ray powder diffraction pattern comprising diffraction peaks at 2θ±0.2° diffraction angles of 7.44°, 11.14°, 11.36°, 14.87°, 15.77°, 16.97°, 17.81°, and 18.61°; furthermore, the free base crystalline form A has an X-ray powder diffraction pattern comprising diffraction peaks at 2θ±0.2° diffraction angles of 3.75°, 7.44°, 11.14°, 11.36°, 11.98°, 12.25°, 14.87°, 15.77°, 16.97°, 17.81°, 18.61°, and 22.36°; still further, the free base crystalline form A has an X-ray powder diffraction pattern comprising diffraction peaks at 2Θ±0.2° diffraction angles of 3.75°, 5.99°, 7.44°, 9.01°, 9.93°, 11.14°, 11.36°, 11.98°, 12.25°, 13.88°, 14.20°, 14.87°, 15.77°, 16.97°, 17.81°, 18.61°, 22.36°, and 24.07°; yet still further, the free base crystalline form A has an X-ray powder diffraction pattern comprising diffraction peaks at 2θ±0.2° diffraction angles of 3.75°, 5.99°, 7.44°, 9.01°, 9.93°, 11.14°, 11.36°, 11.98°, 12.25°, 13.88°, 14.20°, 14.87°, 15.77°, 16.97°, 17.81°, 18.61°, 19.39°, 20.26°, 21.14°, 22.36°, 23.34°, 24.07°, 26.33°, 26.78°, 27.18°, 28.17°, 30.20°, 33.88°, 34.35°, 37.23°, and 37.70°; yet still further, the free base crystalline form A has an XRPD pattern substantially as shown in FIG. 1-1.

In some embodiments, the free base crystalline form A has one, two, or three of the following characteristics:
(1) a TGA curve of the free base crystalline form A showing a weight loss of about 1.28±1% at 150.0±3 °C;
(2) a DSC curve of the free base crystalline form A having a starting point of an endothermic peak at 175.6±3 °C; and
(3) the DSC curve of the free base crystalline form A having an endothermic peak at 176.4±3 °C.

In some embodiments, a TGA/DSC profile of the free base crystalline form A is shown in FIG. 1-2; a ¹H NMR spectrum of the free base crystalline form A is shown in FIG. 1-3.

According to an embodiment of the present disclosure, the free base crystalline form A is an anhydrous crystalline form.

In some embodiments, the free base crystalline form A is in the form of agglomerated needle-like crystals.

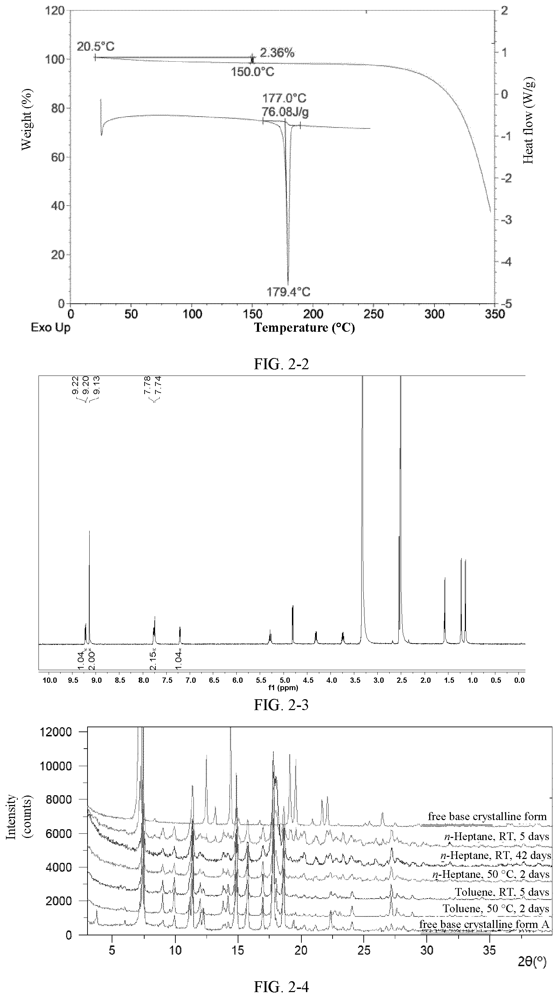

In some embodiments, the present disclosure provides free base crystalline form B of the compound of formula I, wherein the free base crystalline form B has an X-ray powder diffraction pattern comprising diffraction peaks at 2Θ±0.2° diffraction angles of 7.21°, 12.48°, 13.17°, 14.41°, 19.09°, 19.56°, 22.09°, and 26.49°; further, the free base crystalline form B has an X-ray powder diffraction pattern comprising diffraction peaks at 2θ±0.2° diffraction angles of 7.21°, 12.48°, 13.17°, 14.41°, 16.72°, 19.09°, 19.56°, 20.90°, 22.09°, and 26.49°; furthermore, the free base crystalline form B has an X-ray powder diffraction pattern comprising diffraction peaks at 2Θ±0.2° diffraction angles of 7.21°, 8.35°, 12.48°, 13.17°, 14.41°, 15.05°, 16.72°, 17.80°, 18.39°, 19.09°, 19.56°, 20.90°, 21.67°, 22.09°, 22.97°, 25.16°, 26.49°, and 27.49°; still further, the free base crystalline form B has an X-ray powder diffraction pattern comprising diffraction peaks at 2θ±0.2° diffraction angles of 7.21°, 8.35°, 12.48°, 13.17°, 14.41°, 15.05°, 16.72°, 17.80°, 18.39°, 19.09°, 19.56°, 20.90°, 21.67°, 22.09°, 22.97°, 25.16°, 25.45°, 26.49°, 27.49°, 28.66°, 29.10°, 29.35°, 31.71°, 32.00°, 32.85°, 33.70°, 34.23°, 36.78°, 38.26°, and 38.70°; yet still further, the free base crystalline form B has an XRPD pattern substantially as shown in FIG. 2-1.

In some embodiments, the free base crystalline form B has one, two, or three of the following characteristics:
(1) a TGA curve of the free base crystalline form B showing a weight loss of about 2.36±1% at 150.0±3 °C;
(2) a DSC curve of the free base crystalline form B having a starting point of an endothermic peak at 177.0±3 °C; and
(3) the DSC curve of the free base crystalline form B having an endothermic peak at 179.4±3 °C.

In some embodiments, a TGA/DSC profile of the free base crystalline form B is shown in FIG. 2-2; a ¹H NMR spectrum of the free base crystalline form B is shown in FIG. 2-3.

According to an embodiment of the present disclosure, the free base crystalline form B is an anhydrous crystalline form.

In another aspect, the present disclosure provides a pharmaceutically acceptable salt of the compound of formula I, which may be selected from salts formed by the compound of formula I with inorganic acids or organic acids, wherein, for example, the inorganic acids include: hydrochloric acid, sulfuric acid, phosphoric acid, nitric acid, and hydrobromic acid; for example, the organic acids include: maleic acid, L-aspartic acid, fumaric acid, L-tartaric acid, citric acid, D-glucuronic acid, L-malic acid, hippuric acid, D-gluconic acid, DL-lactic acid, succinic acid, L-ascorbic acid, adipic acid, acetic acid, p-toluenesulfonic acid, methanesulfonic acid, benzenesulfonic acid, oxalic acid, 2-hydroxyethanesulfonic acid, malonic acid, gentisic acid, and benzoic acid.

According to a preferred embodiment of the present disclosure, the pharmaceutically acceptable salt of the compound of formula I is a hydrochloride, a maleate, ap-toluenesulfonate, a benzenesulfonate, or a malonate of the compound of formula I.

According to an embodiment of the present disclosure, it can be understood by those skilled in the art that when the compound of formula I forms a salt with an acid, the compound of formula I and the acid may be in a molar ratio of 5:1 to 1:5, such as 3:1, 2:1, 1:1, 1:1.5, 1:2, 1:2.5, or 1:3. Preferably, the compound of formula I and the acid are in a molar ratio of 1:1 or 2:1.

In another aspect, the present disclosure provides a crystalline form of the pharmaceutically acceptable salt of the compound of formula I.

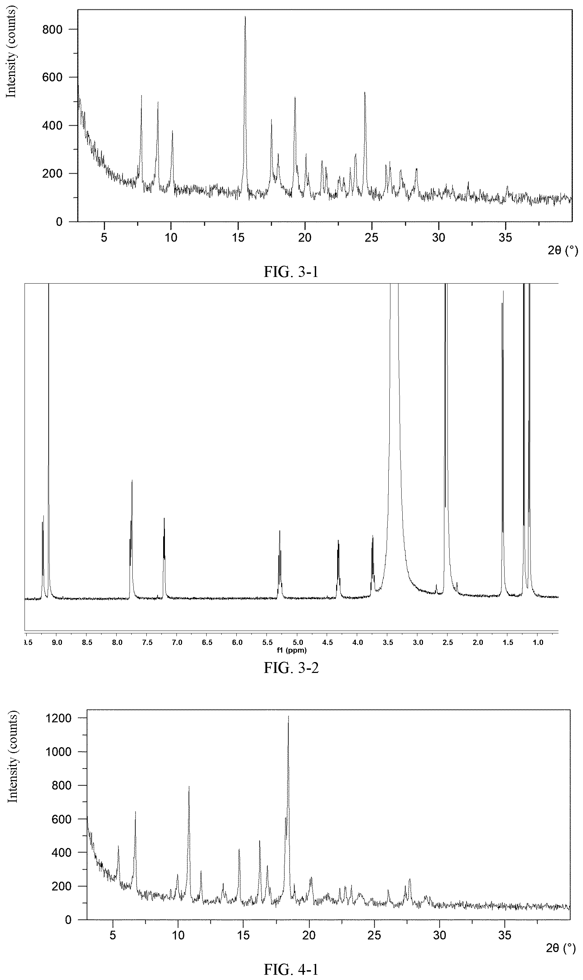

In some embodiments, the present disclosure provides crystalline form A of hydrochloride of the compound of formula I, wherein the crystalline form A of hydrochloride has an X-ray powder diffraction pattern comprising diffraction peaks at 2Θ±0.2° diffraction angles of 7.77°, 9.01°, 10.10°, 15.54°, 17.51°, 19.24°, and 24.49°; further, the crystalline form A of hydrochloride has an X-ray powder diffraction pattern comprising diffraction peaks at 2Θ±0.2° diffraction angles of 7.77°, 9.01°, 10.10°, 15.54°, 17.51°, 18.01°, 19.24°, 20.05°, 21.28°, 23.38°, 23.79°, 24.49°, 26.07°, and 28.33°; furthermore, the crystalline form A of hydrochloride has an X-ray powder diffraction pattern comprising diffraction peaks at 2θ±0.2° diffraction angles of 7.77°, 9.01°, 10.10°, 15.54°, 17.51°, 18.01°, 19.24°, 20.05°, 21.28°, 21.59°, 22.67°, 23.38°, 23.79°, 24.49°, 26.07°, 27.17°, and 28.33°; still further, the crystalline form A of hydrochloride has an XRPD pattern substantially as shown in FIG. 3-1.

In some embodiments, in the crystalline form A of hydrochloride, the compound of formula I and the hydrochloric acid are in a molar ratio of 2:1.

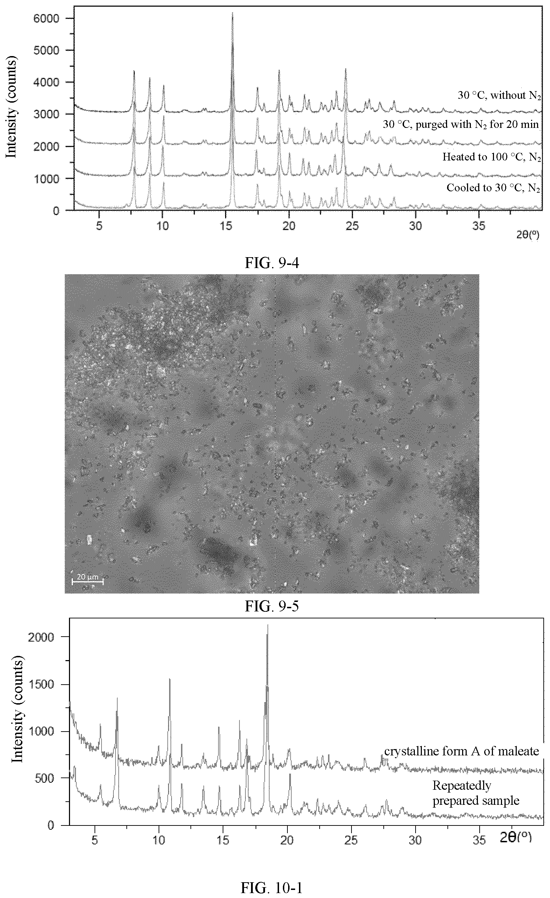

According to an embodiment of the present disclosure, the crystalline form A of hydrochloride has a VT-XRPD pattern substantially as shown in FIG. 9-4.

According to an embodiment of the present disclosure, the crystalline form A of hydrochloride is an anhydrous crystalline form.

In some embodiments, the crystalline form A of hydrochloride has one, two, three, four, five, or six of the following characteristics:
(1) a TGA curve of the crystalline form A of hydrochloride showing a weight loss of about 2.19±1% at 100.0±3 °C;
(2) the TGA curve of the crystalline form A of hydrochloride showing a weight loss of about 3.90±1% in a temperature range of 100.0±3 °C to 160.0±3 °C;
(3) a DSC curve of the crystalline form A of hydrochloride having a starting point of an endothermic peak at 143.6±3 °C;
(4) the DSC curve of the crystalline form A of hydrochloride having an endothermic peak at 157.4±3 °C;
(5) the DSC curve of the crystalline form A of hydrochloride having an endothermic peak at 176.0±3 °C; and
(6) the DSC curve of the crystalline form A of hydrochloride having an endothermic peak at 179.0±3 °C.

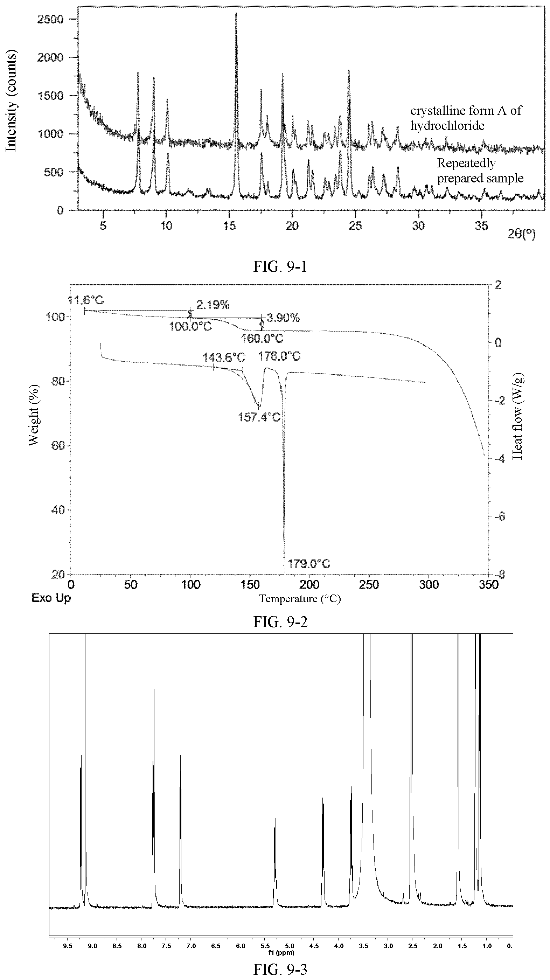

In some embodiments, a TGA/DSC profile of the crystalline form A of hydrochloride is shown in FIG. 9-2; a ¹H NMR spectrum of the crystalline form A of hydrochloride is shown in FIG. 3-2.

In some embodiments, there is no solvent residue in the crystalline form A of hydrochloride.

In some embodiments, the crystalline form A of hydrochloride is in the form of irregular particles.

In some embodiments, the present disclosure provides crystalline form A of maleate of the compound of formula I, wherein the crystalline form A of maleate has an X-ray powder diffraction pattern comprising diffraction peaks at 2Θ±0.2° diffraction angles of 6.73°, 10.84°, 14.68°, 16.26°, 18.23°, and 18.44°; further, the crystalline form A of maleate has an X-ray powder diffraction pattern comprising diffraction peaks at 20±0.2° diffraction angles of 5.43°, 6.73°, 10.84°, 14.68°, 16.26°, 16.82°, 18.23°, and 18.44°; furthermore, the crystalline form A of maleate has an X-ray powder diffraction pattern comprising diffraction peaks at 20±0.2° diffraction angles of 5.43°, 6.73°, 9.95°, 10.84°, 11.75°, 13.50°, 14.68°, 16.26°, 16.82°, 18.23°, 18.44°, 20.17°, 22.79°, 23.22°, 24.00°, 26.07°, 27.72°, and 28.86°; still further, the crystalline form A of maleate has an XRPD pattern substantially as shown in FIG. 4-1.

In some embodiments, in the crystalline form A of maleate, the compound of formula I and the maleic acid are in a molar ratio of 2:1.

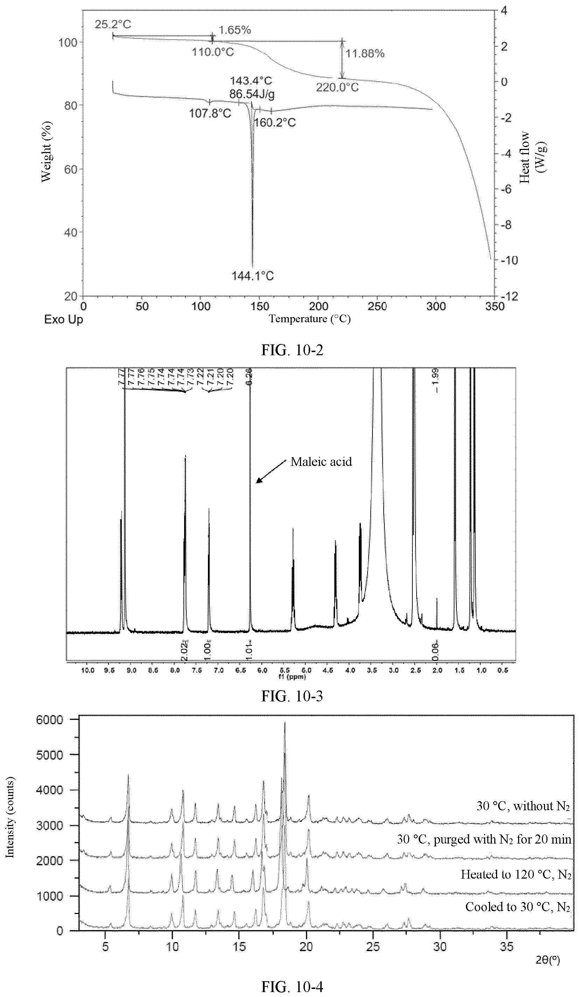

According to an embodiment of the present disclosure, the crystalline form A of maleate has a VT-XRPD pattern substantially as shown in FIG. 10-4.

According to an embodiment of the present disclosure, the crystalline form A of maleate is an anhydrous crystalline form.

In some embodiments, the crystalline form A of maleate has one, two, three, or more of the following characteristics:
(1) a TGA curve of the crystalline form A of maleate showing a weight loss of about 1.65±1% at 110.0±3 °C;
(2) the TGA curve of the crystalline form A of maleate showing a weight loss of about 11.88±1% in a temperature range of 110.0±3 °C to 220.0±3 °C;
(3) a DSC curve of the crystalline form A of maleate having an endothermic peak at 107.8±3 °C;
(4) the DSC curve of the crystalline form A of maleate having a starting point of an endothermic peak at 143.4±3 °C;
(5) the DSC curve of the crystalline form A of maleate having an endothermic peak at 144.1±3 °C; and
(6) the DSC curve of the crystalline form A of maleate having an endothermic peak at 160.2±3 °C.

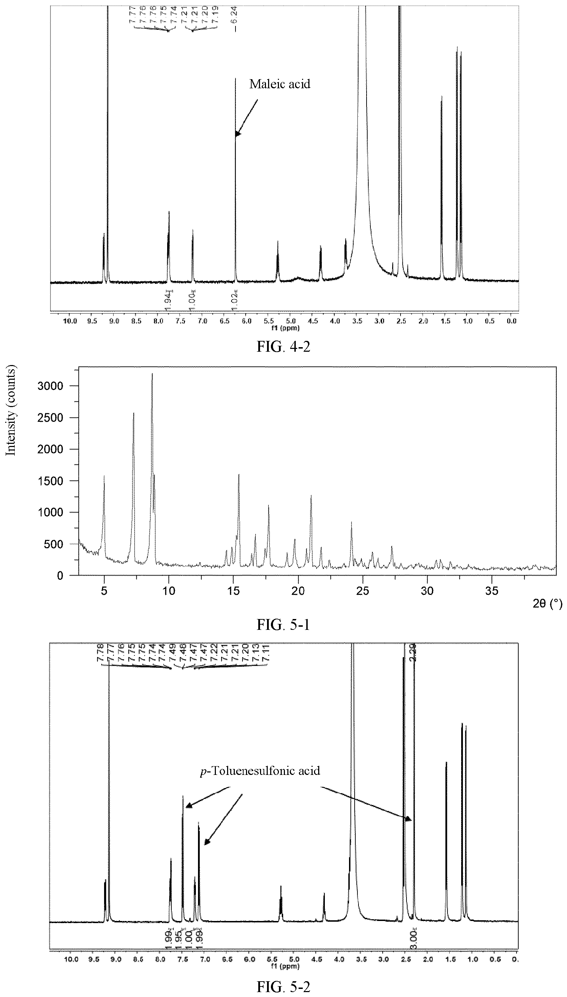

In some embodiments, a TGA/DSC profile of the crystalline form A of maleate is shown in FIG. 10-2; a ¹H NMR spectrum of the crystalline form A of maleate is shown in FIG. 4-2.

In some embodiments, the present disclosure provides crystalline form A of p-toluenesulfonate of the compound of formula I, wherein the crystalline form A of p-toluenesulfonate has an X-ray powder diffraction pattern comprising diffraction peaks at 2Θ±0.2° diffraction angles of 4.99°, 7.26°, 8.70°, 8.87°, 15.40°, 17.73°, 21.01°, and 24.13°; further, the crystalline form A of *p*-toluenesulfonate has an X-ray powder diffraction pattern comprising diffraction peaks at 2Θ±0.2° diffraction angles of 4.99°, 7.26°, 8.70°, 8.87°, 15.20°, 15.40°, 16.68°, 17.73°, 19.71°, 21.01°, and 24.13°; furthermore, the crystalline form A of p-toluenesulfonate has an X-ray powder diffraction pattern comprising diffraction peaks at 2θ=0.2° diffraction angles of 4.99°, 7.26°, 8.70°, 8.87°, 14.45°, 14.88°, 15.20°, 15.40°, 16.41°, 16.68°, 17.45°, 17.73°, 19.16°, 19.71°, 20.66°, 21.01°, 21.76°, 22.41°, 24.13°, 25.76°, 26.18°, and 27.25°; still further, the crystalline form A of p-toluenesulfonate has an X-ray powder diffraction pattern comprising diffraction peaks at 2Θ±0.2° diffraction angles of 4.99°, 7.26°, 8.70°, 8.87°, 14.45°, 14.88°, 15.20°, 15.40°, 16.41°, 16.68°, 17.45°, 17.73°, 19.16°, 19.71°, 20.66°, 21.01°, 21.76°, 22.41°, 24.13°, 25.76°, 26.18°, 27.25°, 27.95°, 29.23°, 30.69°, 31.00°, 31.78°, and 38.41°; yet still further, the crystalline form A of p-toluenesulfonate has an XRPD pattern substantially as shown in FIG. 5-1.

In some embodiments, in the crystalline form A of *p*-toluenesulfonate, the compound of formula I and the p-toluenesulfonic acid are in a molar ratio of 1:1.

According to an embodiment of the present disclosure, the crystalline form A of p-toluenesulfonate is an anhydrous crystalline form.

In some embodiments, the crystalline form A of p-toluenesulfonate has one, two, or three of the following characteristics:
(1) a TGA curve of the crystalline form A of *p*-toluenesulfonate showing a weight loss of about 0.73±1% at 150.0±3 °C;
(2) a DSC curve of the crystalline form A of p-toluenesulfonate having a starting point of an endothermic peak at 157.1±3 °C; and
(3) the DSC curve of the crystalline form A of p-toluenesulfonate having an endothermic peak at 159.2±3 °C.

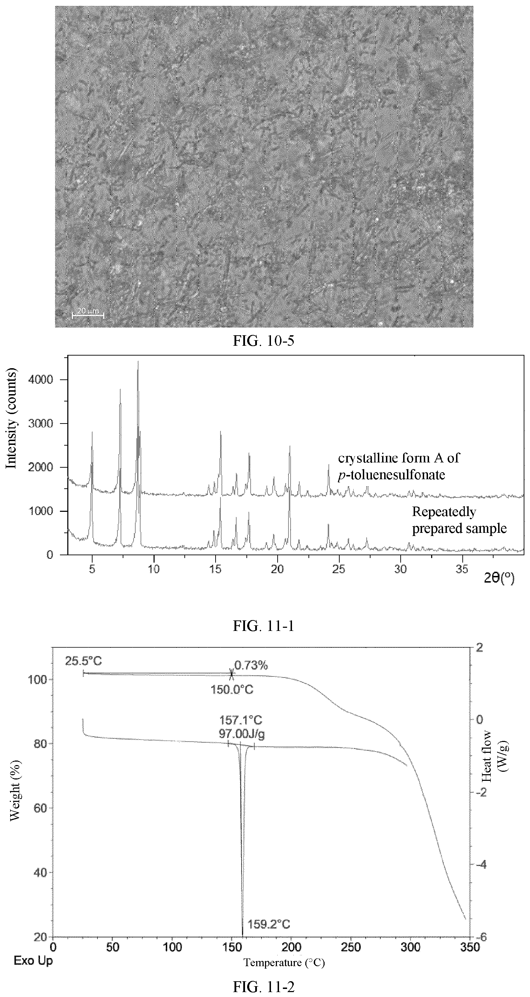

In some embodiments, a TGA/DSC profile of the crystalline form A ofp-toluenesulfonate is shown in FIG. 11-2; a ¹H NMR spectrum of the crystalline form A of *p*-toluenesulfonate is shown in FIG. 5-2.

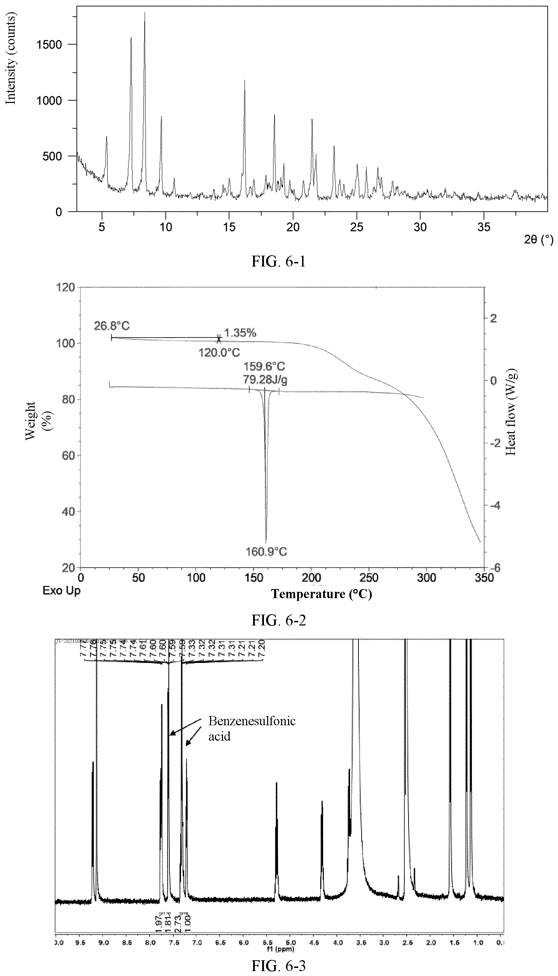

In some embodiments, the present disclosure provides crystalline form A of benzene sulfonate of the compound of formula I, wherein the crystalline form A of benzene sulfonate has an X-ray powder diffraction pattern comprising diffraction peaks at 2Θ±0.2° diffraction angles of 5.36°, 7.28°, 8.34°, 9.64°, 16.20°, 18.55°, and 21.49°; further, the crystalline form A of benzene sulfonate has an X-ray powder diffraction pattern comprising diffraction peaks at 2Θ±0.2° diffraction angles of 5.36°, 7.28°, 8.34°, 9.64°, 16.20°, 18.55°, 19.28°, 21.49°, 21.81°, 23.21°, 25.05°, and 25.74°; furthermore, the crystalline form A of benzene sulfonate has an X-ray powder diffraction pattern comprising diffraction peaks at 2θ±0.2° diffraction angles of 5.36°, 7.28°, 8.34°, 9.64°, 10.66°, 14.55°, 15.00°, 16.20°, 16.93°, 17.85°, 18.55°, 19.28°, 19.74°, 20.80°, 21.49°, 21.81°, 23.21°, 23.68°, 23.98°, 25.05°, 25.74°, 26.65°, and 27.82°; still further, the crystalline form A of benzenesulfonate has an XRPD pattern substantially as shown in FIG. 6-1.

In some embodiments, in the crystalline form A of benzene sulfonate, the compound of formula I and the benzenesulfonic acid are in a molar ratio of 1:1.

According to an embodiment of the present disclosure, the crystalline form A of benzene sulfonate is an anhydrous crystalline form.

In some embodiments, the crystalline form A of benzene sulfonate has one, two, or three of the following characteristics:
(1) a TGA curve of the crystalline form A of benzene sulfonate showing a weight loss of about 1.35±1% at 120.0±3 °C;
(2) a DSC curve of the crystalline form A of benzene sulfonate having a starting point of an endothermic peak at 159.6±3 °C; and
(3) the DSC curve of the crystalline form A of benzenesulfonate having an endothermic peak at 160.9±3 °C. In some embodiments, a TGA/DSC profile of the crystalline form A of benzene sulfonate is shown in FIG. 6-2; a ¹H NMR spectrum of the crystalline form A of benzenesulfonate is shown in FIG. 6-3.

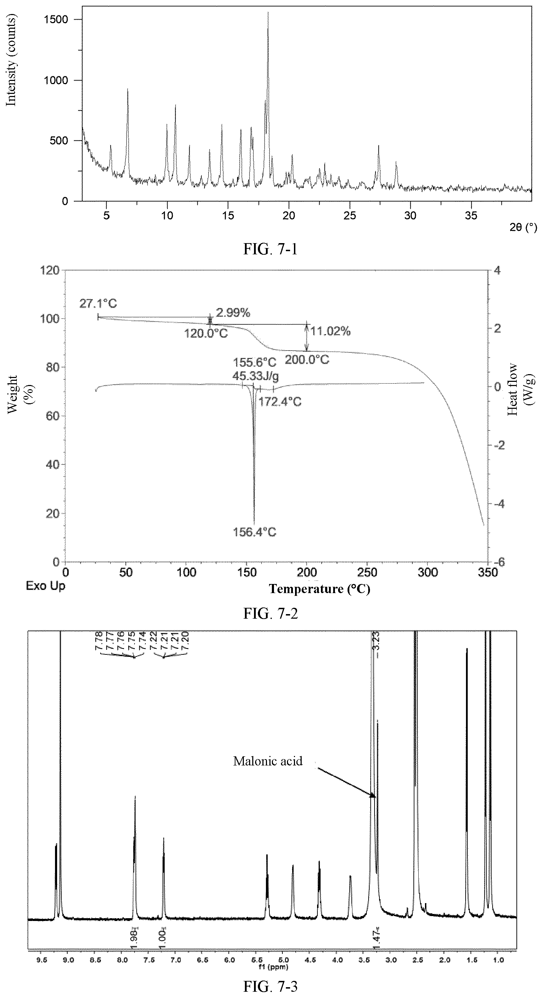

In some embodiments, the present disclosure provides crystalline form A of malonate of the compound of formula I, wherein the crystalline form A of malonate has an X-ray powder diffraction pattern comprising diffraction peaks at 2Θ±0.2° diffraction angles of 6.75°, 9.96°, 10.67°, 14.48°, 16.04°, 16.88°, 18.04°, and 18.29°; further, the crystalline form A of malonate has an X-ray powder diffraction pattern comprising diffraction peaks at 2θ±0.2° diffraction angles of 5.34°, 6.75°, 9.96°, 10.67°, 11.83°, 13.49°, 14.48°, 16.04°, 16.88°, 17.04°, 18.04°, 18.29°, and 27.38°; furthermore, the crystalline form A of malonate has an X-ray powder diffraction pattern comprising diffraction peaks at 2θ=0.2° diffraction angles of 5.34°, 6.75°, 9.96°, 10.67°, 11.83°, 13.49°, 14.48°, 16.04°, 16.88°, 17.04°, 18.04°, 18.29°, 20.27°, 22.57°, 22.95°, 27.38°, and 28.83°; still further, the crystalline form A of malonate has an X-ray powder diffraction pattern comprising diffraction peaks at 2θ±0.2° diffraction angles of 5.34°, 6.75°, 9.96°, 10.67°, 11.83°, 13.49°, 14.48°, 16.04°, 16.88°, 17.04°, 18.04°, 18.29°, 18.63°, 20.27°, 21.57°, 22.57°, 22.95°, 24.11°, 24.83°, 26.02°, 27.38°, and 28.83°; yet still further, the crystalline form A of malonate has an XRPD pattern substantially as shown in FIG. 7-1.

In some embodiments, in the crystalline form A of malonate, the compound of formula I and the malonic acid are in a molar ratio of 2:1.

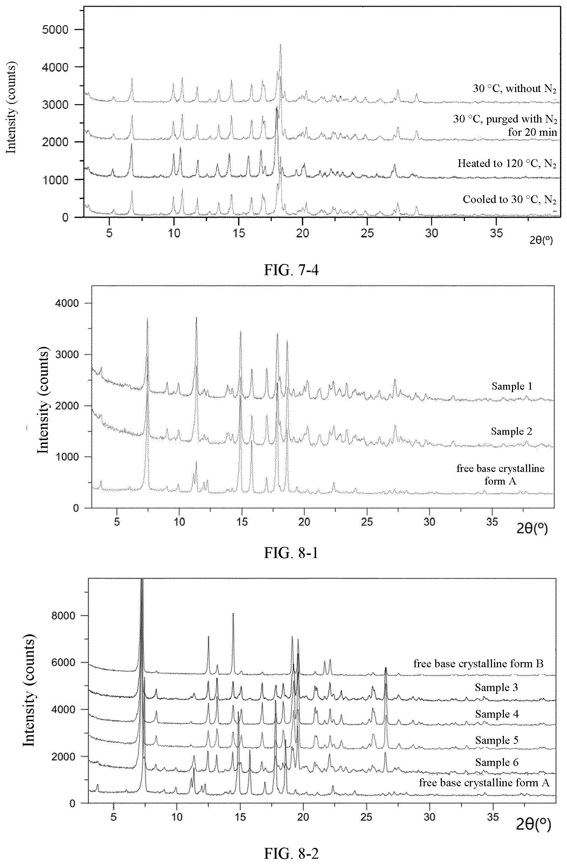

According to an embodiment of the present disclosure, the crystalline form A of malonate has a VT-XRPD pattern substantially as shown in FIG. 7-4.

According to an embodiment of the present disclosure, the crystalline form A of malonate is in an anhydrous crystalline form.

In some embodiments, the crystalline form A of malonate has one, two, three, four, or five of the following characteristics:
(1) a TGA curve of the crystalline form A of malonate showing a weight loss of about 2.99±1% at 120.0±3 °C;
(2) the TGA curve of the crystalline form A of malonate showing a weight loss of about 11.02±1% in a temperature range of 120.0±3 °C to 200.0±3 °C;
(3) a DSC curve of the crystalline form A of malonate having a starting point of an endothermic peak at 155.6±3 °C;
(4) the DSC curve of the crystalline form A of malonate having an endothermic peak at 156.4±3 °C; and
(5) the DSC curve of the crystalline form A of malonate having an endothermic peak at 172.4±3 °C.

In some embodiments, a TGA/DSC profile of the crystalline form A of malonate is shown in FIG. 7-2; a ¹H NMR spectrum of the crystalline form A of malonate is shown in FIG. 7-3.

In another aspect, the present disclosure provides a preparation method for the free base crystalline form A of the compound of formula I, which includes the following methods:
Method 1, comprising adding the compound of formula I to an organic solvent I for dissolution, filtering, and volatilizing at room temperature,
   wherein preferably, the organic solvent I is selected from one or more of acetone, tetrahydrofuran, dichloromethane, acetonitrile, and ethyl acetate;
Method 2, comprising completely dissolving the compound of formula I in an organic solvent II, and dropwise adding an antisolvent to the clarified solution under stirring until a solid precipitates, wherein if no solid precipitates, suspension stirring is adopted; if still no solid precipitates, suspension stirring is performed at a reduced temperature, and then the clarified solution is volatilized at room temperature;
   the organic solvent II may be selected from one or more of methanol, acetone, ethyl acetate, tetrahydrofuran, trichloromethane, *N,N*-dimethylacetamide, and *N*-methylpyrrolidone;
   the antisolvent may be selected from one or more of water, *m*-xylene, n-hexane, cumene, toluene, cyclohexane, *n*-heptane, *n*-pentane, and *p*-cymene, for example, selected from water, a mixture of water and *m*-xylene, a mixture of water and *n*-hexane, a mixture of water and cumene, a mixture of water and toluene, and a mixture of cyclohexane and *p*-cymene;
Method 3, comprising placing an uncovered first sample bottle containing the compound of formula I into a second sample bottle containing a solvent, sealing the second sample bottle, and allowing the second sample bottle to stand at room temperature, wherein the solvent does not cover a mouth of the first sample bottle; the solvent is selected from one or more of ethanol, dichloromethane, acetonitrile, acetone, toluene, *N,N*-dimethylacetamide, and n-hexane;
   preferably, the second sample bottle is allowed to stand for 1-8 days;
Method 4, comprising placing an uncovered first sample bottle containing a solution of the compound of formula I into a second sample bottle containing an antisolvent, sealing the second sample bottle, and allowing the second sample bottle to stand at room temperature, wherein the antisolvent does not cover a mouth of the first sample bottle;
   the solvent in the solution of the compound of formula I is selected from one or more of isopropanol, methyl isobutyl ketone, 1,4-dioxane, and dimethylsulfoxide, and is preferably dimethylsulfoxide;
   the antisolvent is selected from one or more of *n*-pentane, methyl butyl ether, water, and m-xylene, and is preferably a mixture of *n*-pentane and methyl butyl ether, or a mixture of water and *m*-xylene;
Method 5, comprising adding a polymer to a solution of the compound of formula I and allowing the mixture to volatilize at room temperature,
   wherein the solvent in the solution of the compound of formula I is selected from one or more of methanol, 2-butanone, methyl acetate, isopropyl acetate, ethanol, dichloromethane, and 2-methyltetrahydrofuran;
   the polymer is selected from one or more of polyvinylpyrrolidone, polyvinyl alcohol, polyvinyl chloride, polyvinyl acetate, hydroxypropyl methylcellulose, methylcellulose, polycaprolactone, polyethylene glycol, polymethylmethacrylate, sodium alginate, and hydroxyethylcellulose;
   preferably, the polymer is selected from a mixed polymer A or a mixed polymer B, wherein the mixed polymer A consists of polyvinylpyrrolidone, polyvinyl alcohol, polyvinyl chloride, polyvinyl acetate, hydroxypropyl methylcellulose, and methylcellulose, and the mixed polymer B consists of polycaprolactone, polyethylene glycol, polymethylmethacrylate, sodium alginate, and hydroxyethylcellulose;
   preferably, the polymer and the compound of formula I are in a mass ratio of 1:(5-20);
Method 6, comprising placing an uncovered first sample bottle containing the compound of formula I into a second sample bottle containing a saturated salt solution or water, sealing the second sample bottle, and allowing the second sample bottle to stand at room temperature, wherein the solvent does not cover a mouth of the first sample bottle;
   preferably, the saturated salt solution is a saturated inorganic salt solution, which may be selected from, for example, a saturated potassium acetate solution, a saturated potassium carbonate solution, a saturated sodium bromide solution, or a saturated potassium bromide solution;
   preferably, the humidity of the system is 15%-100% RH;
Method 7, comprising stirring a suspension of the compound of formula I under temperature cycling, performing centrifugation, and collecting a solid,
   wherein a solvent in the suspension is selected from one or more of n-heptane, methyl butyl ether, anisole, dicyclohexylamine, acetone, ethanol, ethyl acetate, methylcyclohexane, trichloromethane, 2-butanone, m-xylene, and water, for example, selected from n-heptane, methyl butyl ether, anisole, a mixture of butanone and water, a mixture of ethanol and water, a mixture of ethyl acetate and methylcyclohexane, a mixture of trichloromethane and n-heptane, or a mixture of 2-butanone and m-xylene;
   preferably, conditions for the temperature cycling include: 50 °C to 5 °C, 0.1-0.5 °C/min, and at least 2 cycles;
Method 8, comprising completely dissolving the compound of formula I in a good solvent, and dropwise adding an antisolvent to the clarified solution under stirring until a solid precipitates, wherein if no solid precipitates, suspension stirring is adopted; if still no solid precipitates, suspension stirring is performed at a reduced temperature, and then the clarified solution is volatilized at room temperature;
   the good solvent may be selected from one or more of ethanol, ethyl acetate, 2-methyltetrahydrofuran, 2-butanone, acetonitrile, dichloromethane, and 1,4-dioxane;
   the antisolvent is selected from one or more of n-heptane, tetrahydrofuran, and water;
Method 9, comprising placing a turbid solution of the compound of formula I at room temperature with magnetic stirring, performing centrifugation, and collecting a solid,
   wherein a solvent in the turbid solution is selected from one or more of isobutanol, methyl tert-butyl ether, cyclohexane, toluene, isopropyl acetate, water, methylcyclohexane, tetrahydrofuran, n-pentane, acetone, isopropanol, cyclopentyl methyl ether, methanol, *p*-cymene, dichloromethane, n-heptane, acetonitrile, 1,4-dioxane, and *N*-methylpyrrolidone; for example, the solvent of the turbid solution is selected from isobutanol, methyl *tert*-butyl ether, cyclohexane, toluene, a mixture of isopropyl acetate and toluene, water, methylcyclohexane, a mixture of tetrahydrofuran and *n*-pentane, a mixture of acetone and isopropanol, a mixture of isopropanol and cyclopentyl methyl ether, a mixture of dichloromethane and n-heptane, a mixture of acetonitrile and water, a mixture of 1,4-dioxane and water, or a mixture of *N*-methylpyrrolidone and water;
   preferably, the magnetic stirring is performed at a rotation speed of 700-1200 rpm;
Method 10, comprising weighing the compound of formula I into an HPLC bottle, adding a solvent into the HPLC bottle, heating and stirring for equilibration, then filtering, and collecting a supernatant; placing the supernatant into a biological incubator, cooling from 50 °C to 5 °C at 0.05 °C/min and then maintaining the temperature at 5 °C, transferring the clarified solution to -20 °C and maintaining the temperature, collecting a precipitated solid, and transferring the sample without solid precipitation to the room temperature for volatilization,
   wherein the solvent is selected from one or more of isopropanol, anisole, isopropyl acetate, tetrahydrofuran, and water;
   the target temperature of the heating is 45-55 °C, preferably 50 °C; and
Method 11, comprising stirring a suspension of the compound of formula I, performing centrifugation, and collecting a solid,
   wherein a solvent in the suspension is selected from one or more of n-butanol(n-BuOH), toluene, isopropyl ether, methylcyclohexane, cumene, anisole, water, petroleum ether, dicyclohexylamine, 2-methyltetrahydrofuran, *n*-hexane, 2-butanone, isopropyl acetate, trichloromethane, *m*-xylene, tetrahydrofuran, methyl isobutyl ketone, cyclopentyl methyl ether, and benzyl alcohol, for example, a mixture of *n*-BuOH and toluene, isopropyl ether, methylcyclohexane, cumene, anisole, water, PET, dicyclohexylamine, a mixture of 2-butanone and cumene, a mixture of isopropyl acetate and toluene, a mixture of trichloromethane and *m*-xylene, a mixture of isopropyl acetate and water, a mixture of tetrahydrofuran and water, a mixture of methyl isobutyl ketone and cyclopentyl methyl ether, or a mixture of benzyl alcohol and toluene;
   the suspension is stirred at 45-55 °C, preferably 50 °C;
   preferably, the stirring is magnetic stirring, for example, performed at a rotation speed of 700-1200 rpm.

In another aspect, the present disclosure provides a preparation method for the free base crystalline form B of the compound of formula I, comprising the following steps:
dissolving free base crystalline form A of the compound of formula I in 1,4-dioxane, and then performing gas-liquid diffusion in an n-hexane atmosphere to give the free base crystalline form B of the compound of formula I.

The present disclosure further provides a preparation method for the pharmaceutically acceptable salt of the compound of formula I, comprising the following step: mixing the compound of formula I or free base crystalline form A of the compound of formula I with a salt-forming reagent (e.g., a corresponding acid) in a suitable solvent to give a mixture.

In some embodiments, the preparation method further comprises the steps of: stirring or slurring the mixture, separating out a solid, and vacuum drying to give the pharmaceutically acceptable salt of the compound of formula I, wherein preferably, the stirring, the slurring, and the vacuum drying are performed at room temperature.

In some embodiments, the preparation method further comprises the step of increasing the supersaturation of the mixture (e.g., by addition of an antisolvent).

In some embodiments, the solvent is selected from one or a mixture of more of ethanol, heptane, ethyl acetate, MTBE, acetonitrile, water, and acetone.

In yet another aspect, the present disclosure provides a pharmaceutical composition, comprising one or more of the free base crystalline form (e.g., free base crystalline form A or free base crystalline form B) of the compound of formula I and the pharmaceutically acceptable salt (including the crystalline form thereof) of the compound of formula I.

In some embodiments, the pharmaceutical composition further comprises a pharmaceutically acceptable excipient or carrier.

In yet another aspect, the present disclosure provides use of the free base crystalline form (e.g., free base crystalline form A or free base crystalline form B) of the compound of formula I, the pharmaceutically acceptable salt (including the crystalline form thereof) of the compound of formula I, or the pharmaceutical composition for the manufacturing of a medicament for the treatment and/or prevention of a P2X3-related disease.

According to an embodiment of the present disclosure, a better and more effective clinical therapeutic medicament or regimen may be provided to a patient in need thereof by using the free base crystalline form (e.g., free base crystalline form A or free base crystalline form B) of the compound of formula I, the pharmaceutically acceptable salt (including the crystalline form thereof) of the compound of formula I, or the pharmaceutical composition of the present disclosure.

The present disclosure further provides a method for treating and/or preventing a P2X3-related disease, wherein the method comprises administering to a patient a therapeutically effective dose of a pharmaceutical formulation comprising the crystalline form of the compound of formula I as described above, the pharmaceutically acceptable salt of the compound of formula I as described above, the crystalline form of the salt as described above, or the pharmaceutical composition as described above, preferably comprising the free base crystalline form (e.g., free base crystalline form A or free base crystalline form B) of the compound of formula I, the pharmaceutically acceptable salt (including the crystalline form thereof) of the compound of formula I, or the pharmaceutical composition as described above.

In some preferred embodiments, the pharmaceutically acceptable salt of the compound of formula I includes salts formed by the compound of formula I with acids selected from: hydrochloric acid, sulfuric acid, maleic acid, L-aspartic acid, phosphoric acid, fumaric acid, L-tartaric acid, citric acid, D-glucuronic acid, L-malic acid, hippuric acid, D-gluconic acid, DL-lactic acid, succinic acid, L-ascorbic acid, adipic acid, acetic acid, p-toluenesulfonic acid, methanesulfonic acid, benzenesulfonic acid, oxalic acid, 2-hydroxyethanesulfonic acid, malonic acid, gentisic acid, and benzoic acid.

Preferably, the pharmaceutically acceptable salt of the compound of formula I includes the crystalline form A of hydrochloride, the crystalline form A of maleate, the crystalline form A of p-toluenesulfonate, the crystalline form A of benzene sulfonate, the crystalline form A of malonate, or any combination of salts thereof.

According to an embodiment of the present disclosure, the P2X3-related disease includes: pain, genitourinary system diseases, or respiratory system diseases.

Preferably, the pain includes: inflammatory pain, surgical pain, visceral pain, dental pain, premenstrual pain, central pain, burn-induced pain, migraine, or cluster headache; preferably, the genitourinary system diseases include: urinary incontinence, overactive bladder, dysuria, cystitis, endometriosis, and endometriosis-associated pain; preferably, the respiratory system diseases include: cough, idiopathic pulmonary fibrosis (IPF), and chronic obstructive pulmonary disease (COPD); preferably, the cough includes subacute or chronic cough, treatment-resistant cough, idiopathic chronic cough, post-viral cough, iatrogenic cough, and cough associated with respiratory diseases (e.g., COPD, asthma, and bronchospasm).

According to an embodiment of the present disclosure, the P2X3-related disease includes at least one selected from the following diseases: chronic cough, especially refractory chronic cough (RCC) and unexplained chronic cough (UCC).

The present disclosure further provides a quality detection method for the crystalline form of the compound of formula I or the pharmaceutically acceptable salt thereof, comprising the following step: detecting the content of the crystalline form by using high-performance liquid chromatography, wherein mobile phases adopted in the high-performance liquid chromatography include a mobile phase A and a mobile phase B; the mobile phase A is an aqueous solution of formic acid (FA) and acetonitrile (ACN); the mobile phase B is acetonitrile.
preferably, the mobile phase A is an aqueous solution of 0.05%-0.15% formic acid and 2%-7% acetonitrile, illustratively an aqueous solution of 0.1% formic acid and 5% acetonitrile.

Preferably, the quality detection method comprises: a purity detection method, a solubility detection method, and a stability detection method.

Preferably, the high-performance liquid chromatography adopts gradient elution.

Preferably, the flow rate of the mobile phases is 1±0.2 mL/min; the gradient elution is performed for 5-60 min, more preferably 10-30 min.

Preferably, in the gradient elution, the mobile phase A and the mobile phase B are in a volume ratio of 1:9 to 9:1.

### Definitions and Explanations of Terms

Although various terms and phrases used herein have general meanings known to those skilled in the art, the present disclosure intends to provide a more detailed illustration and explanation of these terms and phrases herein. The meanings of the terms and phrases mentioned herein shall prevail in the event of any inconsistency with those well known.

Unless otherwise stated, a numerical range set forth in the specification and claims shall be construed as at least including each specific integer value within the range. For example, two or more represent 2, 3, 4, 5, 6, 7, 8, 9, 10, or more. When certain numerical ranges are defined or understood as "numbers", it shall be construed as including both endpoints of the range, each integer within the range, and each decimal within the range. For example, "a number of 0-10" shall be construed as including not only each of integers 0, 1, 2, 3, 4, 5, 6, 7, 8, 9, and 10, but also at least the sums of each integer and 0.1, 0.2, 0.3, 0.4, 0.5, 0.6, 0.7, 0.8, or 0.9.

When "about" a certain numerical value is recited in the specification and claims, it shall be construed as including the numerical value itself, as well as numerical values within a range around the numerical value that is acceptable in the art, for example, numerical values within the range of ±15% of the numerical value, numerical values within the range of ±10% of the numerical value, numerical values within the range of ±5% of the numerical value, and the like. For example, about 10 represents that it includes a numerical value within the range of 10±1.5, i.e., within the range of 8.5 to 11.5; a numerical value within the range of 10±1.0, i.e., within the range of 9.0 to 11.0; and a numerical value within the range of 10±0.5, i.e., within the range of 9.5 to 10.5.

The salts and polymorphs of the compound of formula I of the present disclosure can be used in combination with other active ingredients, as long as they do not produce other adverse effects, such as allergic reactions. The term "composition" as used herein is intended to encompass a product comprising specified ingredients in specified amounts, as well as any product that results, directly or indirectly, from combination of the specified ingredients in the specified amounts.

The term "patient" refers to any animal including mammals, preferably mice, rats, other rodents, rabbits, dogs, cats, pigs, cattle, sheep, horses, or primates, and most preferably humans.

The term "therapeutically effective dose" refers to the amount of the active compound or drug that causes a biological or medical response that researchers, veterinarians, physicians, or other clinicians are looking for in tissues, systems, animals, individuals, or humans, including one or more of the following effects: (1) disease prevention: for example, the prevention of a disease, disorder or condition in an individual who is susceptible to the disease, disorder or condition but has not yet experienced or exhibited the pathology or symptoms of the disease; (2) disease inhibition: for example, the inhibition of a disease, disorder or condition in an individual who is experiencing or exhibiting the pathology or symptoms of the disease, disorder or condition.(i.e., the prevention of the further development of the pathology and/or symptoms); and (3) disease alleviation: for example, the alleviation of a disease, disorder or condition in an individual who is experiencing or exhibiting the pathology or symptoms of the disease, disorder or condition (i.e., the reverse of the pathology and/or symptoms).

The term "pharmaceutically acceptable" means that a prescription component or an active ingredient does not unduly and adversely affect a general therapeutic target's health.

The term "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present disclosure and with each other, without significantly diminishing the pharmaceutical efficacy of the compound. Examples of pharmaceutically acceptable excipients or carriers include cellulose and its derivatives (e.g., sodium carboxymethylcellulose, sodium ethylcellulose, cellulose acetate, and the like), gelatin, talc, solid lubricants (e.g., stearic acid and magnesium stearate), calcium sulfate, vegetable oil (e.g., soybean oil, sesame oil, peanut oil, olive oil, and the like), polyols (e.g., propylene glycol, glycerol, mannitol, sorbitol, and the like), emulsifiers, wetting agents (e.g., sodium dodecyl sulfate), colorants, flavoring agents, stabilizers, antioxidants, preservatives, pyrogen-free water, etc. The pharmaceutical composition may be specifically formulated in solid or liquid form for oral administration, for parenteral injection, or for rectal administration. The pharmaceutical composition can be formulated into a variety of dosage forms to facilitate administration, such as oral formulations (e.g., tablets, capsules, solutions, or suspensions), injectable formulations (e.g., injectable solutions or suspensions, or injectable dry powders that can be used immediately after addition of a pharmaceutical vehicle before injection).

When used for the therapeutic and/or prophylactic purposes described above, the total daily amount of the salt, polymorph and pharmaceutical composition of the compound of formula I of the present disclosure will be determined by an attending physician within the scope of sound medical judgment. For any specific patient, the specific therapeutically effective dose level will depend upon a variety of factors including the disorder being treated and the severity of the disorder, the activity of the specific compound employed, the specific composition employed, the age, body weight, general health condition, gender, and diet of the patient, the time of administration, route of administration and excretion rate for the specific compound employed, the duration of the treatment, the drugs used in combination or simultaneously with the specific compound employed, and similar factors well known in the medical field. For example, it is known in the art to start with a dose of a compound at a level below that required to achieve a desired therapeutic effect and then gradually increase the dose until the desired therapeutic effect is achieved.

The term "API" refers to a free base, i.e., the compound of formula I.

### Beneficial Effects

1) The present disclosure provides a crystalline form of a compound of formula I, which has good medicinal properties, wherein the free base crystalline form A has relatively low hygroscopicity, good solubility, and good physicochemical stability, is a thermodynamically stable crystalline form at room temperature and 50 °C, and is beneficial for storage and quality stability of the medicament and further druggability.
2) The present disclosure screens and obtains pharmaceutically acceptable salts of the compound of formula I through optimization tests, and further obtains crystalline form products of the salts, such as crystalline form A of hydrochloride, crystalline form A of maleate, crystalline form A of *p*-toluenesulfonate, crystalline form A of benzenesulfonate, and crystalline form A of malonate, wherein the crystalline form A of *p*-toluenesulfonate has almost no hygroscopicity and possesses good physicochemical stability, higher solubility in biological vehicles, and better druggability value.

### BRIEF DESCRIPTION OF THE DRAWINGS

FIG. 1-1 is an XRPD pattern of free base crystalline form A of a compound of formula I;
FIG. 1-2 is a TGA/DSC profile of the free base crystalline form A of the compound of formula I;
FIG. 1-3 is a ¹H NMR spectrum of the free base crystalline form A of the compound of formula I;
FIG. 1-4 is a PLM image of the free base crystalline form A of the compound of formula I;
FIG. 2-1 is an XRPD pattern of free base crystalline form B of the compound of formula I;
FIG. 2-2 is a TGA/DSC profile of the free base crystalline form B of the compound of formula I;
FIG. 2-3 is a ¹H NMR spectrum of the free base crystalline form B of the compound of formula I;
FIG. 2-4 is an XRPD overlay of competitive suspension test samples of free base crystalline forms A/B;
FIG. 3-1 is an XRPD pattern of crystalline form A of hydrochloride of the compound of formula I;
FIG. 3-2 is a ¹H NMR spectrum of the crystalline form A of hydrochloride of the compound of formula I;
FIG. 4-1 is an XRPD pattern of crystalline form A of maleate of the compound of formula I;
FIG. 4-2 is a ¹H NMR spectrum of the crystalline form A of maleate of the compound of formula I;
FIG. 5-1 is an XRPD pattern of crystalline form A of *p*-toluenesulfonate of the compound of formula I;
FIG. 5-2 is a ¹H NMR spectrum of the crystalline form A of *p*-toluenesulfonate of the compound of formula I;
FIG. 6-1 is an XRPD pattern of crystalline form A of benzenesulfonate of the compound of formula I;
FIG. 6-2 is a TGA/DSC profile of the crystalline form A of benzenesulfonate of the compound of formula I;
FIG. 6-3 is a ¹H NMR spectrum of the crystalline form A of benzene sulfonate of the compound of formula I;
FIG. 7-1 is an XRPD pattern of crystalline form A of malonate of the compound of formula I;
FIG. 7-2 is a TGA/DSC profile of the crystalline form A of malonate of the compound of formula I;
FIG. 7-3 is a ¹H NMR spectrum of the crystalline form A of malonate of the compound of formula I;
FIG. 7-4 is a VT-XRPD pattern of crystalline form A of malonate of the compound of formula I;
FIG. 8-1 is an XRPD overlay of samples 1 and 2;
FIG. 8-2 is an XRPD overlay of samples 3, 4, 5 and 6;
FIG. 9-1 is an XRPD pattern of crystalline form A of hydrochloride of the compound of formula I prepared repeatedly;
FIG. 9-2 is a TGA/DSC profile of the crystalline form A of hydrochloride of the compound of formula I prepared repeatedly;
FIG. 9-3 is a ¹H NMR spectrum of the crystalline form A of hydrochloride of the compound of formula I prepared repeatedly;
FIG. 9-4 is a VT-XRPD pattern of the crystalline form A of hydrochloride of the compound of formula I prepared repeatedly;
FIG. 9-5 is a PLM image of the crystalline form A of hydrochloride of the compound of formula I prepared repeatedly;
FIG. 10-1 is an XRPD pattern of crystalline form A of maleate of the compound of formula I prepared repeatedly;
FIG. 10-2 is a TGA/DSC profile of the crystalline form A of maleate of the compound of formula I prepared repeatedly;
FIG. 10-3 is a ¹H NMR spectrum of the crystalline form A of maleate of the compound of formula I prepared repeatedly;
FIG. 10-4 is a VT-XRPD pattern of the crystalline form A of maleate of the compound of formula I prepared repeatedly;
FIG. 10-5 is a PLM image of the crystalline form A of maleate of the compound of formula I prepared repeatedly;
FIG. 11-1 is an XRPD pattern of crystalline form A of *p*-toluenesulfonate of the compound of formula I prepared repeatedly;
FIG. 11-2 is a TGA/DSC profile of the crystalline form A of *p*-toluenesulfonate of the compound of formula I prepared repeatedly;

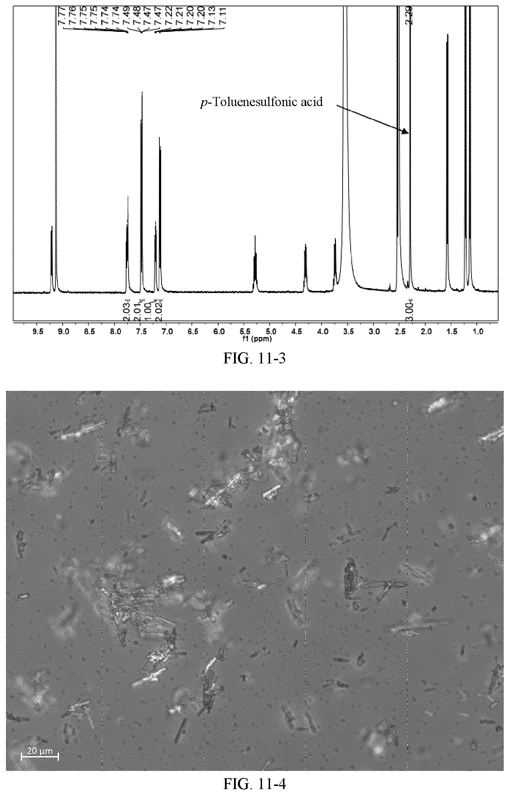

FIG. 11-3 is a ¹H NMR spectrum of the crystalline form A of *p*-toluenesulfonate of the compound of formula I prepared repeatedly;
FIG. 11-4 is a PLM image of the crystalline form A of *p*-toluenesulfonate of the compound of formula I prepared repeatedly;

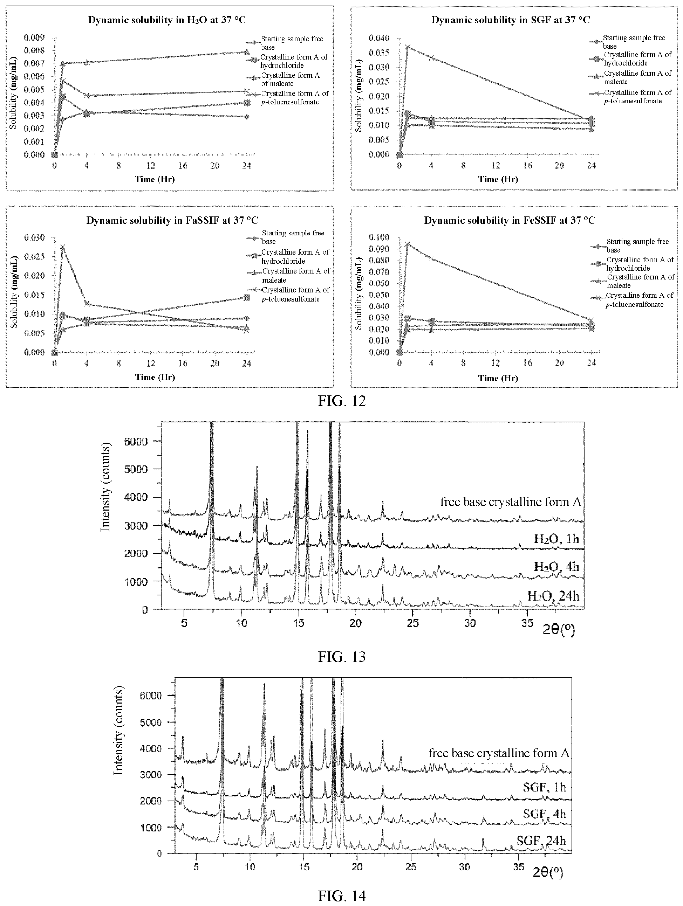

FIG. 12 is a dynamic solubility curve at 37 °C;
FIG. 13 is an XRPD overlay of solubility samples of the free base crystalline form A in HzO;
FIG. 14 is an XRPD overlay of solubility samples of the free base crystalline form A in SGF;

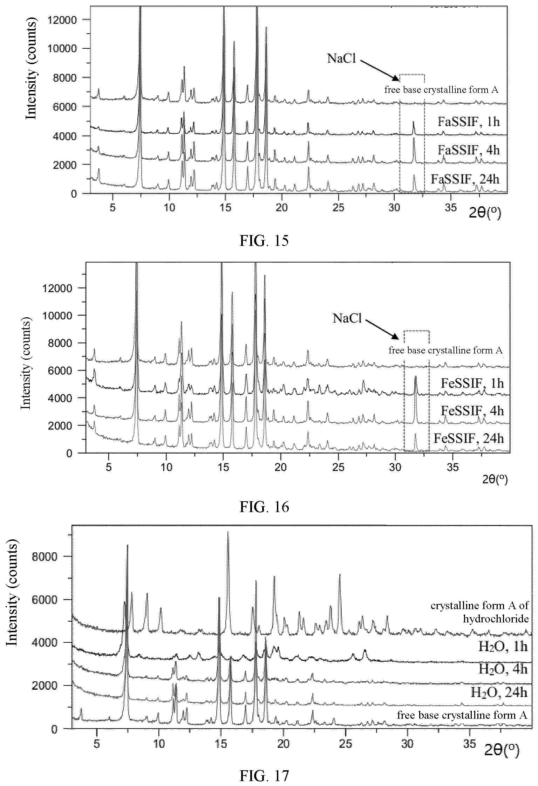

FIG. 15 is an XRPD overlay of solubility samples of the free base crystalline form A in FaSSIF;
FIG. 16 is an XRPD overlay of solubility samples of the free base crystalline form A in FeSSIF;
FIG. 17 is an XRPD overlay of solubility samples of the crystalline form A of hydrochloride in HzO;

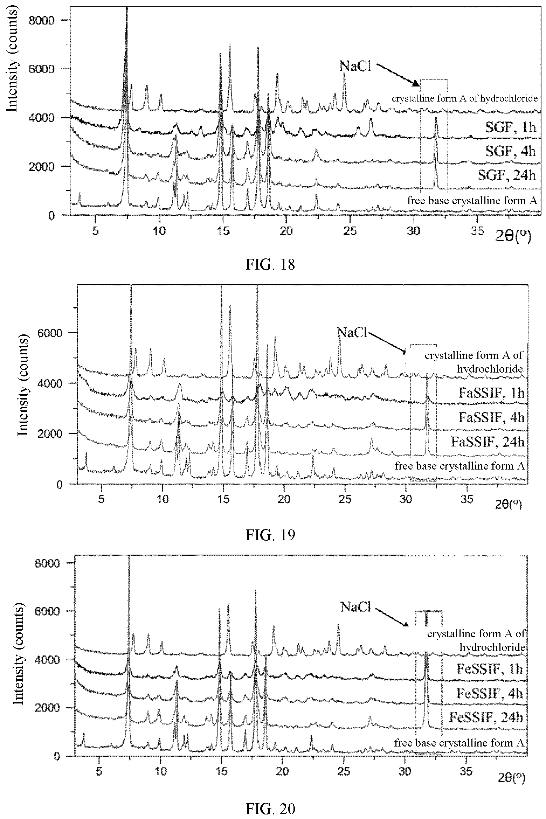

FIG. 18 is an XRPD overlay of solubility samples of the crystalline form A of hydrochloride in SGF;
FIG. 19 is an XRPD overlay of solubility samples of the crystalline form A of hydrochloride in FaSSIF;
FIG. 20 is an XRPD overlay of solubility samples of the crystalline form A of hydrochloride in FeSSIF;

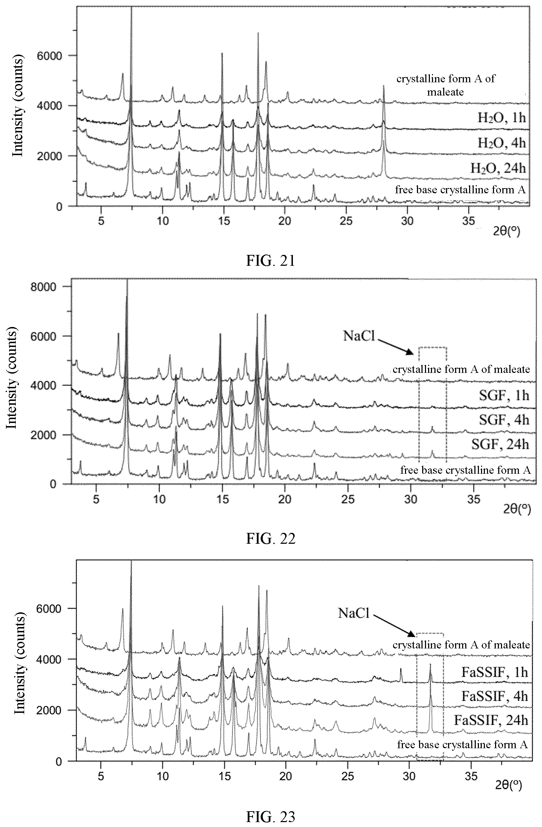

FIG. 21 is an XRPD overlay of solubility samples of the crystalline form A of maleate in HzO;
FIG. 22 is an XRPD overlay of solubility samples of the crystalline form A of maleate in SGF;
FIG. 23 is an XRPD overlay of solubility samples of the crystalline form A of maleate in FaSSIF;

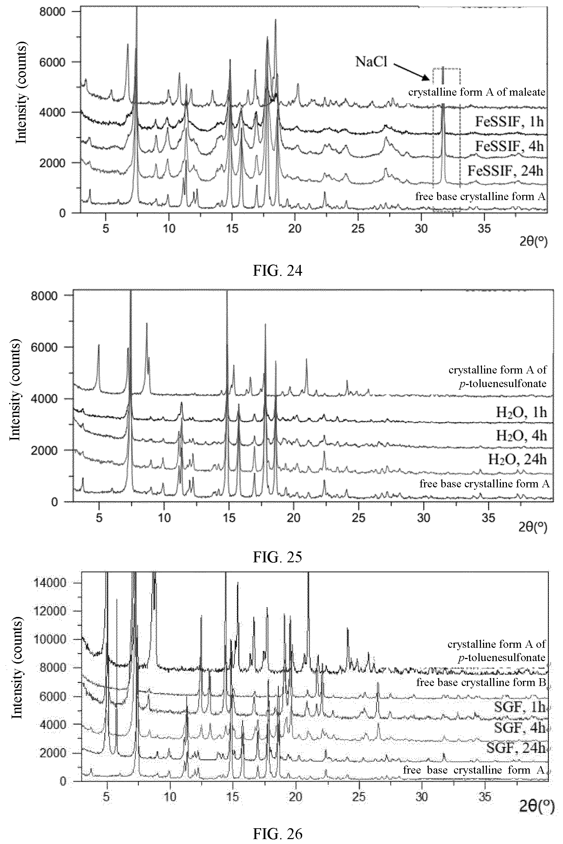

FIG. 24 is an XRPD overlay of solubility samples of the crystalline form A of maleate in FeSSIF;
FIG. 25 is an XRPD overlay of solubility samples of the crystalline form A of *p*-toluenesulfonate in HzO;
FIG. 26 is an XRPD overlay of solubility samples of the crystalline form A of *p*-toluenesulfonate in SGF;

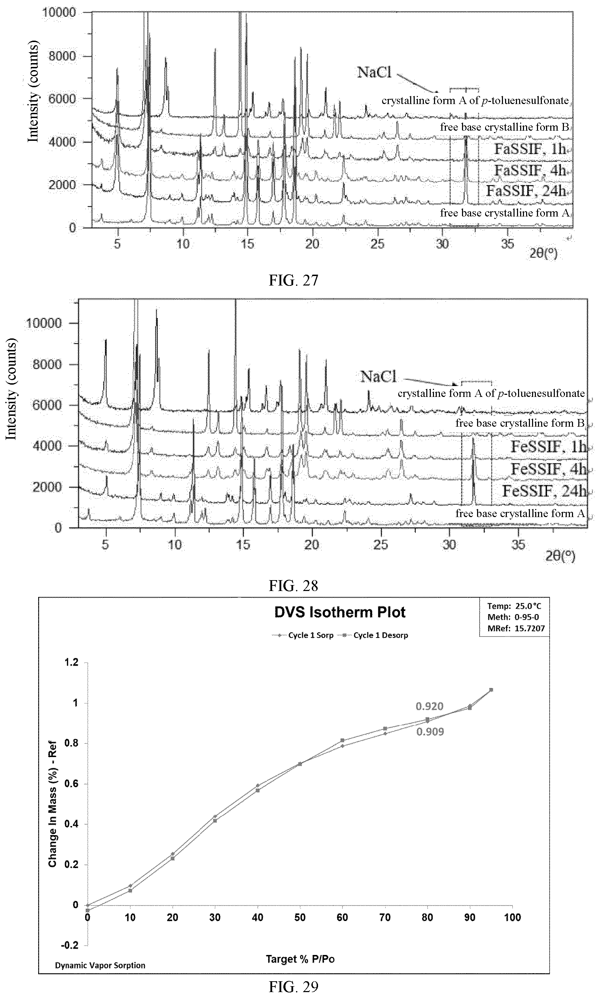

FIG. 27 is an XRPD overlay of solubility samples of the crystalline form A of *p*-toluenesulfonate in FaSSIF;
FIG. 28 is an XRPD overlay of solubility samples of the crystalline form A of *p*-toluenesulfonate in FeSSIF;
FIG. 29 is a DVS profile of the free base crystalline form A;

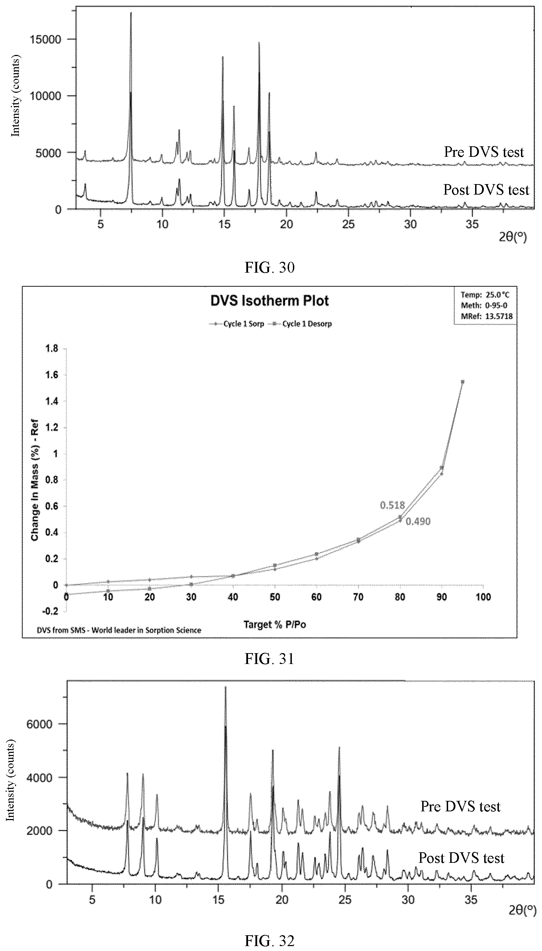

FIG. 30 is an XRPD overlay of the free base crystalline form A before and after DVS test;
FIG. 31 is a DVS profile of the crystalline form A of hydrochloride;
FIG. 32 is an XRPD overlay of the crystalline form A of hydrochloride before and after DVS test;

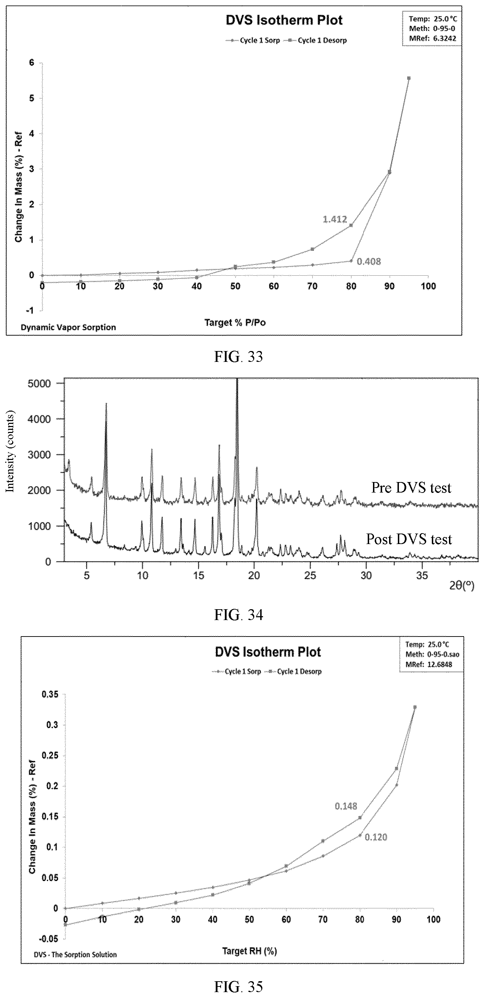

FIG. 33 is a DVS profile of the crystalline form A of maleate;
FIG. 34 is an XRPD overlay of the crystalline form A of maleate before and after DVS test;
FIG. 35 is a DVS profile of the crystalline form A of*p*-toluenesulfonate;

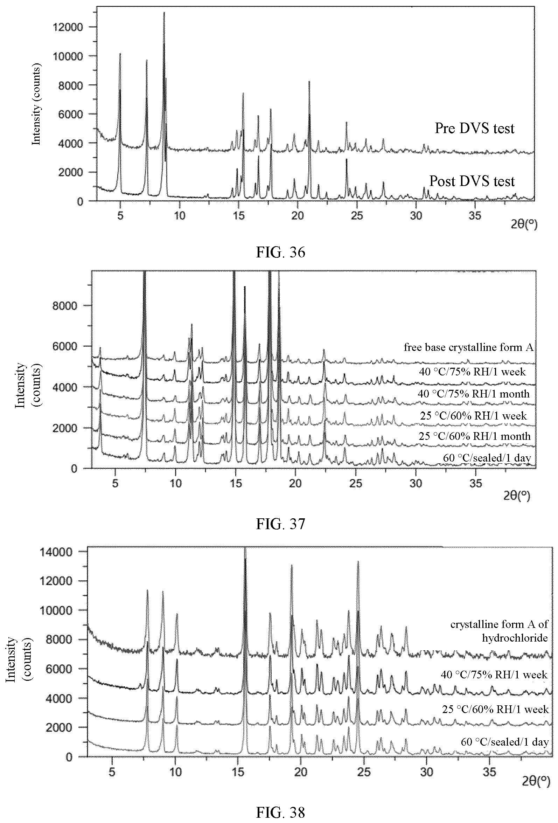

FIG. 36 is an XRPD overlay of the crystalline form A of *p*-toluenesulfonate before and after DVS test;
FIG. 37 is an XRPD overlay of stability evaluation samples of the free base crystalline form A;
FIG. 38 is an XRPD overlay of stability evaluation samples of the crystalline form A of hydrochloride;

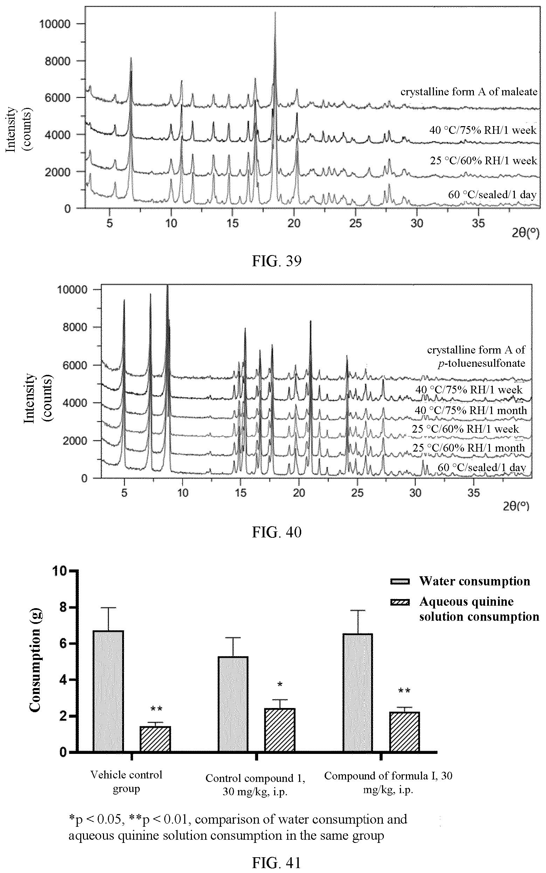

FIG. 39 is an XRPD overlay of stability evaluation samples of the crystalline form A of maleate;
FIG. 40 is an XRPD overlay of stability evaluation samples of the crystalline form A of*p*-toluenesulfonate;
FIG. 41 shows the test results of water intake/aqueous quinine intake ratio for animals given the corresponding compounds;

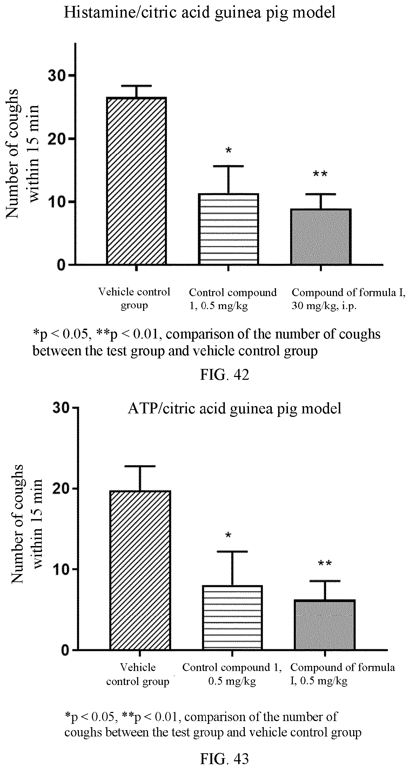

FIG. 42 shows the test results of the number of coughs stimulated by histamine/citric acid in guinea pigs after administration of the corresponding compounds; and
FIG. 43 shows the test results of the number of coughs stimulated by ATP/citric acid in guinea pigs after administration of the corresponding compounds.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the contents described above of the present disclosure are encompassed within the protection scope of the present disclosure.

Unless otherwise stated, the starting materials and reagents used in the following examples are all commercially available products or can be prepared by using known methods.

The instruments and detection methods adopted in the present disclosure are as follows:

### I. X-ray powder diffraction (XRPD)

XRPD patterns were acquired on an X-ray powder diffraction analyzer manufactured by PANalytacal, and the scanning parameters are shown in Table A-1 below.

**Table A-1. XRPD test parameters**

| **Model** | **X' Pert3/Empyrean** | **Empyrean (VT)** |
|---|---|---|
| X-ray | Cu, Kα, | Cu, Kα, |
| | Kα1 (Å):1.540598, | Kα1 (Å):1.540598, |
| | Kα2 (Å):1.544426 | Kα2 (Å):1.544426 |
| | Kα2/Kα1 intensity ratio:0.50 | Kα2/Kα1 intensity ratio:0.50 |
| X-ray light tube settings | 45 kV, 40 mA | 45 kV, 40 mA |
| Divergence slit | 1/8° | 1/8° |
| Scanning mode | Continuous | Continuous |
| Scanning range (°2Theta) | 3-40 | 3-40 |
| Scanning time per step (s) | 46.7 | 46.7 |
| Scanning step length (°2Theta) | 0.0263 | 0.0167 |
| Test time | ~5 min | ~10 min |

### II. Thermogravimetric analysis (TGA) and differential scanning calorimetry (DSC)

TGA and DSC profiles were acquired on a TA 5500 thermogravimetric analyzer and a TA 2500 differential scanning calorimeter, respectively, and the test parameters are listed in Table A-2 below.

**Table A-2. DSC and TGA test parameters**

| **Parameter** | **TGA** | **DSC** |
|---|---|---|
| Method | Linear heating | Linear heating |
| Sample tray | Aluminum tray, open | Aluminum tray, with/without cover |
| Temperature range | Room temperature-setting endpoint temperature | 25 °C-setting endpoint temperature |
| Purging rate (°C/min) | 10 | 10 |
| Protective gas | Nitrogen | Nitrogen |

### III. Dynamic vapor sorption (DVS)

Dynamic vapor sorption (DVS) curves were acquired on a DVS IntrInsic from Surface Measurement Systems (SMS). The relative humidity at 25 °C was corrected with the deliquescence points of LiCl, Mg(NO₃)₂, and KCl. The DVS test parameters are listed in Table A-3 below.

**Table A-3. DVS test parameters**

| **Parameter** | **Set value** |
|---|---|
| Temperature | 25 °C |
| Sample amount | 10-20 mg |
| Protective gas and flow rate | N₂, 200 mL/min |
| dm/dt | 0.002%/min |
| Minimum dm/dt equilibration time | 10 min |
| Maximum equilibration time | 180 min |
| RH range | 0%RH-95%RH |
| RH gradient | 10%RH (0%RH - 90%RH & 90%RH - 0%RH) |
| | 5%RH (90%RH - 95%RH & 95%RH - 90%RH) |

### IV. Polarizing microscope (PLM)

The polarizing microscope photographs were taken at room temperature using a Zeiss Axio Scope.A1 microscope.

### V. Liquid-state nuclear magnetic resonance (¹H NMR)

The liquid-state nuclear magnetic resonance spectra were acquired on a Bruker 400M nuclear magnetic resonance spectrometer with DMSO-*d₆* as a solvent.

### VI. High-performance liquid chromatography (HPLC)

In the test, the purity, dynamic solubility and stability were tested using an Agilent 1260 high-performance liquid chromatograph, the molar ratio of salt-forming ions was tested using ion chromatography, and the analytical conditions are shown in Tables A-4 and A-5 below.

**Table A-4. Test conditions for high-performance liquid chromatography**

| **Liquid chromatograph** | **Agilent 1260/1290 detector** | | | |
|---|---|---|---|---|
| Chromatographic column | ACE 3 C18-AR, 4.6 mm/150 mm/3 µm | | | |
| Mobile phase | A: 0.1% FA & 5% ACN in H₂O | | | |
| | B: acetonitrile | | | |
| Elution gradient | Solubility | | Purity | |
| | **Time (min)** | **%B** | **Time (min)** | **%B** |
| | 0.0 | 10 | 0.0 | 10 |
| | 6.0 | 90 | 24.0 | 90 |
| | 7.0 | 90 | 27.0 | 90 |
| | 7.1 | 10 | 27.1 | 10 |
| | 10.0 | 10 | 30.0 | 10 |
| Run time | 10 min | | 30 min | |
| Flow rate of mobile phase | | 1.0 mL/min | | |
| Injection volume | | 10 µL | | |
| Detection wavelength | | UV at 220&254 nm | | |
| Column temperature | | 30 °C | | |
| Injector temperature | | RT | | |
| Diluent | | Acetonitrile | | |

**Table A-5. Test conditions for ion chromatography**

| **Ion chromatograph** | **ThermoFisher ICS-1100** |
|---|---|
| Chromatographic column | IonPac AS18 Analytical Column, 250*4 mm |
| Mobile phase | 25 mM NaOH |
| Injection volume | 25 µL |
| Flow rate | 1.0 mL/min |
| Temperature | 35 °C |
| Column temperature | 35 °C |
| Current | 80 mA |
| Run time | Cl⁻: 7 min |

The reagents used in the present disclosure are shown in Table A-6 below.

**Table A-6. Names of solvents used in the tests**

| **In English** | **In Chinese** | **In English** | **In Chinese** |
|---|---|---|---|
| MeOH | 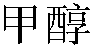 | 1,4-dioxane | 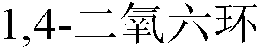 |
| EtOH | 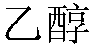 | ACN | 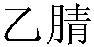 |
| IPA | 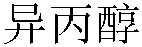 | DCM | 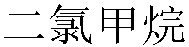 |
| Acetone | 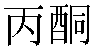 | CHCl₃ | 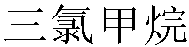 |
| MIBK | 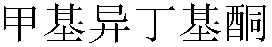 | Toluene | 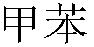 |
| EtOAc | 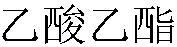 | n-Heptane | 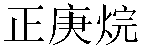 |
| IPAc | 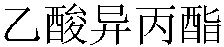 | DMSO | 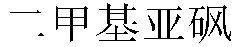 |
| MTBE | 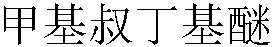 | DMAc | 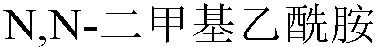 |
| THF | 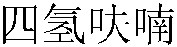 | NMP | 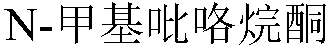 |
| 2-MeTHF | 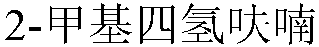 | DMF | 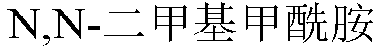 |
| CPME | 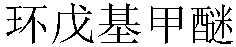 | H₂O | 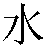 |
| m-Xylene | 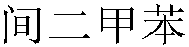 | n-Pentane | 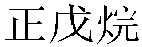 |
| p-Cymene | 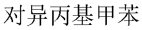 | Cyclohexane | 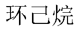 |
| n-Hexane | 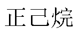 | MEK | 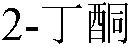 |
| Methyl butyl ether | 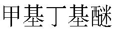 | Cumene | 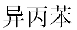 |
| Methylcyclohexane | 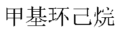 | Anisole | 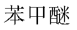 |
| Butyl acetate | 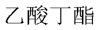 | Dicyclohexylamine | 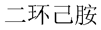 |
| TFE | 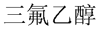 | IBA | 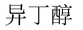 |
| Methyl butyl ether | 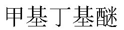 | PTE | 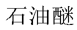 |
| Methyl acetate | 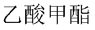 | Benzyl alcohol | 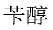 |

### Example 1. Synthesis of Intermediate A-1

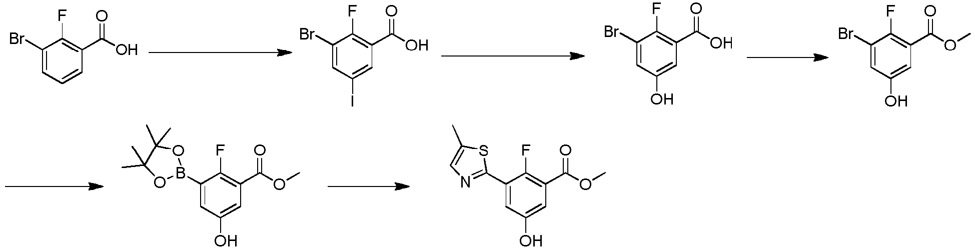

### Step 1: Synthesis of 3-bromo-2-fluoro-5-iodobenzoic acid

3-Bromo-2-fluorobenzoic acid (10 g, 45.7 mmol) was dissolved in concentrated sulfuric acid (40 mL), and NIS (10.27 g, 45.7 mmol) was added in batches at 0 °C. The mixture was stirred at room temperature for three hours. Ice water (200 mL) was then added to quench the reaction, and the mixture was filtered. The filter cake was washed five times with water (200 mL) and dried under vacuum to give 3-bromo-2-fluoro-5-iodobenzoic acid (10.9 g, white solid, yield: 69.2%).

### Step 2: Synthesis of 3-bromo-2-fluoro-5-hydroxybenzoic acid

Cuprous oxide (0.656 g, 4.74 mmol) was added to a solution of 3-bromo-2-fluoro-5-iodobenzoic acid (10.9 g, 31.6 mmol) and sodium hydroxide (6.32 g, 158 mmol) in water (100 mL), and the mixture was allowed to react at 100 °C overnight. The reaction mixture was then cooled to room temperature and filtered. The filtrate was adjusted to pH = 1 with a 2 M hydrochloric acid solution and extracted with ethyl acetate (60 mL×3). The organic phase was concentrated to dryness to give 3-bromo-2-fluoro-5-hydroxybenzoic acid, (7.2 g, yellow solid, yield: 96.8%).

### Step 3: Synthesis of methyl 3-bromo-2-fluoro-5-hydroxybenzoate

Thionyl chloride (10.9 g, 91.8 mmol) was added to a solution of 3-bromo-2-fluoro-5-hydroxybenzoic acid (7.2 g, 30.6 mmol) in methanol (120 mL), and the mixture was stirred at 55 °C for 16 h. The reaction mixture was then concentrated under reduced pressure to remove the solvent, giving the compound methyl 3-bromo-2-fluoro-5-hydroxybenzoate as a solid (3.1 g, yield: 40.8%), which was used in the next step without further purification.

### Step 4: Synthesis of methyl 2-fluoro-5-hydroxy-3-(4,4,5,5-tetramethyl-1,3,2-dioxaborolan-2-yl)benzoate

Methyl 3-bromo-2-fluoro-5-hydroxybenzoate (3.1 g, 12.45 mmol), bis(pinacolato)diboron (3.48 g, 13.69 mmol), and potassium acetate (3.67 g, 37.3 mmol) were dissolved in 1,4-dioxane (50 mL), and the solution was degassed with a stream of nitrogen for 2 min. Pd(dppf)Cl₂ (0.455 g, 0.622 mmol)) was added, and the resulting solution was degassed with a stream of nitrogen for another 2 min. The reaction mixture was then stirred at 100 °C for 16 h, filtered, and concentrated under vacuum. The residue was separated and purified by a silica gel column to give methyl 2-fluoro-5-hydroxy-3-(4,4,5,5-tetramethyl-1,3,2-dioxaborolan-2-yl) benzoate (3.4 g, white solid, yield: 92%).

### Step 5: Synthesis of methyl 2-fluoro-5-hydroxy-3-(5-methylthiazol-2-yl)benzoate (intermediate A-1)

Pd(dppf)Cl₂ (1.260 g, 1.722 mmol) was added to a mixed solution of methyl 2-fluoro-5-hydroxy-3-(4,4,5,5-tetramethyl-1,3,2-dioxaborolan-2-yl)benzoate (3.4 g, 11.48 mmol), 2-bromo-5-methylthiazole (2.453 g, 13.78 mmol), and potassium carbonate (3.81 g, 27.6 mmol) in THF (30 mL) and water (10 mL) at room temperature, and the mixture was purged three times with nitrogen under vacuum and then allowed to react at 90 °C for 16 h. The reaction mixture was diluted with water (30 mL) and extracted with ethyl acetate (40 mL × 3). The organic phase was concentrated to dryness, and the residue was separated and purified by a silica gel column to give methyl 2-fluoro-5-hydroxy-3-(5-methylthiazol-2-yl)benzoate (intermediate A-1, 1.41 g, yellow solid, yield: 45.9%). LC-MS, M/Z: 268.2 [M+H]⁺.

### Example 2. Preparation of Compound of Formula I

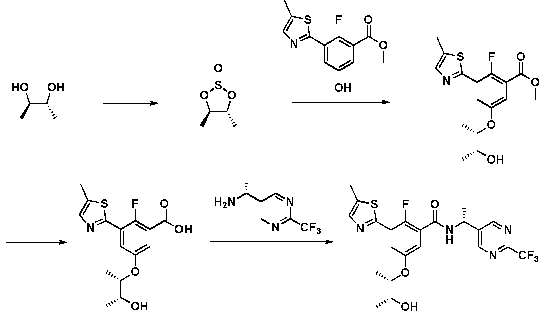

### Step 1: Synthesis of (4R,5R)-4,5-dimethyl-1,3,2-dioxathiolane-2-oxide

(2R,3R)-Butane-2,3-diol (2 g, 22.19 mmol) and pyridine (3.86 g, 48.8 mmol) were dissolved in dry tetrahydrofuran (20 mL). The reaction temperature was adjusted to 0-5 °C, and thionyl chloride (2.9 g, 24.41 mmol) was slowly added. The mixture was warmed to room temperature and stirred for 16 h. Water (30 mL) was added to quench the reaction, and 20 mL of ethyl acetate was added, followed by liquid separation. The organic phase was washed with saturated ammonium chloride (20 mL) and then with a saturated aqueous sodium chloride solution (20 mL), dried over anhydrous sodium sulfate, and concentrated under reduced pressure to give (4R,5R)-4,5-dimethyl-1,3,2-dioxathiolane-2-oxide (2.4 g, colorless liquid, yield: 79%).

¹H NMR (400 MHz, Chloroform-d) δ 4.63 (dq, J=9.0, 6.1 Hz, 1H), 4.07 (dq, J=9.0, 6.1 Hz, 1H), 1.52 (d, J=6.2 Hz, 3H), 1.43 (d, J=6.1 Hz, 3H).

### Step 2: Synthesis of methyl 2-fluoro-5-(((2S,3R)-3-hydroxybutan-2-yl)oxy)-3-(5-methylthiazol-2-yl) benzoate

Under nitrogen atmosphere, cesium carbonate (1.22 g, 3.74 mmol) was added to a solution of methyl 2-fluoro-5-hydroxy-3-(5-methylthiazol-2-yl)benzoate (intermediate A-1, 500 mg, 1.871 mmol) in *N,N*-dimethylformamide (5 mL), and the mixture was stirred at room temperature for 30 min.

(4R,5R)-4,5-Dimethyl-1,3,2-dioxathiolane-2-oxide (382 mg, 2.81 mmol) was added, and the mixture was warmed to 80 °C and allowed to react for 16 h. The reaction mixture was then cooled to room temperature and concentrated under reduced pressure to dryness. Chloroform (20 mL) and a 4 M sulfuric acid solution (20 mL) were added, and the mixed solution was stirred at 70 °C for 5 h, followed by liquid separation. The aqueous phase was adjusted to pH 7-8 with sodium bicarbonate and extracted with dichloromethane (20 mL × 3). The organic phases were combined, dried over anhydrous sodium sulfate, and concentrated, and the residue was purified by a silica gel column (petroleum ether:ethyl acetate (V/V) = 2:1) to give methyl 2-fluoro-5-(((2*S*,3*R*)-3-hydroxybutan-2-yl)oxy)-3-(5-methylthiazol-2-yl)benzoate (380 mg, white solid, yield: 60%).

LC-MS, M/Z: 340.1 [M+H]⁺.

Step 3: Synthesis of 2-fluoro-5-(((2*S*,3*R*)-3-hydroxybutan-2-yl)oxy)-3-(5-methylthiazol-2-yl)benzoic acid Methyl 2-fluoro-5-(((2*S*,3*R*)-3-hydroxybutan-2-yl)oxy)-3-(5-methylthiazol-2-yl)benzoate (200 mg, 0.589 mmol) was dissolved in methanol (4 mL), and then lithium hydroxide monohydrate (70.7 mg, 1.765 mmol) and water (0.4 mL) were added. The mixture was stirred and allowed to react at room temperature for 16 h. The reaction mixture was then concentrated directly to dryness, and water (5 mL) was added. The resulting mixture was adjusted to pH 2-3 with a 1 M aqueous hydrochloric acid solution. The aqueous phase was extracted with dichloromethane (5 mL × 3). The organic phases were combined, dried over anhydrous sodium sulfate, and concentrated to give 2-fluoro-5-(((2S,3R)-3-hydroxybutan-2-yl)oxy)-3-(5-methylthiazol-2-yl)benzoic acid (190 mg, white solid, yield: 99%).

LC-MS, M/Z: 326.1 [M+H]⁺.

### Step 4:

### 2-fluoro-5-(((2S,3R)-3-hydroxybutan-2-yl)oxy)-3-(5-methylthiazol-2-yl)-N-((R)-1-(2-(trifluoromethyl)pyrimi din-5-yl)ethyl)benzamide

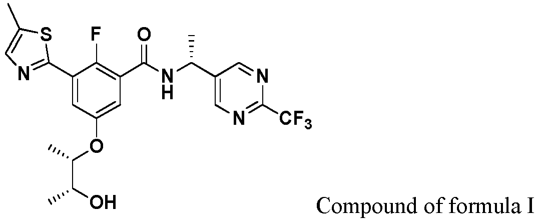

Under nitrogen atmosphere, 2-fluoro-5-(((2S,3R)-3-hydroxybutan-2-yl)oxy)-3-(5-methylthiazol-2-yl)benzoic acid (190 mg, 0.584 mmol), (*R*)-1-(2-(trifluoromethyl)pyrimidin-5-yl)ethan-1-amine hydrochloride (160 mg, 0.701 mmol), *N,N*-diisopropylethylamine (226 mg, 1.752 mmol), and *N,N-*dimethylformamide (5 mL) were sequentially added into a reaction bottle, and the mixture was cooled to about 0 °C. A solution of 1-propylphosphoric anhydride in *N,N-*dimethylformamide (50%, 557 mg, 0.876 mmol) was then added dropwise. After the addition, the mixture was warmed to room temperature and allowed to react for 16 h. A saturated sodium bicarbonate solution (5 mL) was then added to quench the reaction, and the mixture was extracted with ethyl acetate (10 mL × 3). The organic phases were combined, washed with saturated brine (30 mL × 2), dried over anhydrous sodium sulfate, and concentrated, and the residue was separated and purified by a silica gel plate (petroleum ether:ethyl acetate (V/V) = 1:1) to give 2-fluoro-5-(((2*S*,3*R*)-3-hydroxybutan-2-yl)oxy)-3-(5-methylthiazol-2-yl)-*N*-((*R*)-1-(2-(trifluoromethyl)pyrimi din-5-yl)ethyl)benzamide as a white solid (the compound of formula I, 110 mg, yield: 37.8%).

¹H NMR (400 MHz, CDCl₃) δ 8.94 (s, 2H), 7.86 (dd, J=5.9, 3.3 Hz, 1H), 7.63-7.58 (m, 1H), 7.50 (dd, J=5.8, 3.4 Hz, 1H), 7.10 (dd, J=12.3, 6.5 Hz, 1H), 5.38 (ddd, J=7.7, 4.4, 1.5 Hz, 1H), 4.40 (qd, J=6.3, 3.3 Hz, 1H), 4.01 (ddd, J=6.5, 4.8, 3.3 Hz, 1H), 2.56 (d, J=1.2 Hz, 3H), 2.07 (d, J=4.9 Hz, 1H), 1.72 (d, J=7.1 Hz, 3H), 1.26 (d, J=6.3 Hz, 3H), 1.23 (d, J=6.5 Hz, 3H).

LC-MS, M/Z: 499.1 [M+H]⁺.

### Example 3. Preparation of Free Base Crystalline Form A of Compound of Formula I

About 20 mg of a sample of the compound of formula I was weighed into an HPLC glass vial, and 0.5 mL of toluene was added. The resulting suspension was magnetically stirred (1000 rpm) at room temperature for about 4 days and then centrifuged (10000 rpm, 2 min) to collect a solid. The solid was then subjected to an XRPD test, as shown in FIG. 1-1. Based on the test results, the solid product was designated as free base crystalline form A. The TGA/DSC results (FIG. 1-2) show that the sample had a weight loss of 1.28% when heated to 150 °C, and one endothermic peak at 175.6 °C (onset temperature). The ¹H NMR results of the free base crystalline form A are shown in FIG. 1-3. The PLM image (FIG. 1-4) shows that the sample was in the form of agglomerated needle-like crystals.

The XRPD analysis data of the free base crystalline form A of the compound of formula I obtained are shown in Table 3-1 below:

**Table 3-1. XRPD diffraction peak data of free base crystalline form A**

| **Peak number** | **2θ [°]** | **Relative intensity [%]** |
|---|---|---|
| 1 | 3.7545 | 9.64 |
| 2 | 5.9913 | 4.63 |
| 3 | 7.4445 | 100.00 |
| 4 | 9.0114 | 5.32 |
| 5 | 9.9288 | 7.15 |
| 6 | 11.1445 | 15.10 |
| 7 | 11.3633 | 23.38 |
| 8 | 11.9815 | 8.28 |
| 9 | 12.2464 | 9.84 |
| 10 | 13.8765 | 2.53 |
| 11 | 14.2009 | 4.26 |
| 12 | 14.8698 | 70.17 |
| 13 | 15.7733 | 39.36 |
| 14 | 16.9721 | 11.87 |
| 15 | 17.8126 | 81.62 |
| 16 | 18.6098 | 48.69 |
| 17 | 19.3928 | 5.09 |
| 18 | 20.2599 | 2.75 |
| 19 | 21.1401 | 2.78 |
| 20 | 22.3632 | 8.68 |
| 21 | 23.3375 | 1.73 |
| 22 | 24.0748 | 4.12 |
| 23 | 26.3329 | 1.47 |
| 24 | 26.7755 | 2.41 |
| 25 | 27.1851 | 3.26 |
| 26 | 28.1700 | 2.41 |
| 27 | 30.2016 | 0.59 |
| 28 | 33.8787 | 0.87 |
| 29 | 34.3489 | 2.54 |
| 30 | 37.2273 | 1.82 |
| 31 | 37.7008 | 1.64 |

### Example 4. Preparation of Free Base Crystalline Form B of Compound of Formula I

The free base crystalline form A of the compound of formula I was completely dissolved in 1,4-dioxane and then subjected to gas-liquid diffusion in an n-hexane atmosphere. The solid was then air-dried at room temperature to give free base crystalline form B.

The XRPD pattern of the free base crystalline form B is shown in FIG. 2-1. The TGA/DSC results (FIG. 2-2) show that the sample had a weight loss of 2.36% when heated to 150 °C, and one endothermic peak at 177.0 °C (onset temperature). The ¹H NMR results of the free base crystalline form B are shown in FIG. 2-3.

The XRPD analysis data of the free base crystalline form B of the compound of formula I obtained are shown in Table 4-1 below:

**Table 4-1. XRPD diffraction peak data of free base crystalline form B**

| **Peak number** | **2θ [°]** | **Relative intensity [%]** |
|---|---|---|
| 1 | 7.2097 | 100.00 |
| 2 | 8.3529 | 3.38 |
| 3 | 12.4780 | 14.58 |
| 4 | 13.1747 | 12.05 |
| 5 | 14.4127 | 12.91 |
| 6 | 15.0523 | 4.87 |
| 7 | 16.7188 | 6.47 |
| 8 | 17.8033 | 2.47 |
| 9 | 18.3882 | 3.46 |
| 10 | 19.0885 | 15.72 |
| 11 | 19.5650 | 30.76 |
| 12 | 20.8990 | 6.02 |
| 13 | 21.6739 | 3.31 |
| 14 | 22.0914 | 10.60 |
| 15 | 22.9694 | 2.81 |
| 16 | 25.1648 | 2.13 |
| 17 | 25.4470 | 3.96 |
| 18 | 26.4880 | 11.92 |
| 19 | 27.4871 | 2.77 |
| 20 | 28.6571 | 0.93 |
| 21 | 29.0977 | 1.10 |
| 22 | 29.3475 | 1.31 |
| 23 | 31.7143 | 1.05 |
| 24 | 31.9962 | 1.21 |
| 25 | 32.8508 | 2.20 |
| 26 | 33.6989 | 1.04 |
| 27 | 34.2326 | 2.22 |
| 28 | 36.7810 | 0.69 |
| 29 | 38.2619 | 0.40 |
| 30 | 38.6957 | 1.33 |

In order to confirm the thermodynamic transformation relationship between the anhydrous free base crystalline forms A and B under different temperature conditions, competitive suspension tests in n-heptane and toluene at room temperature and 50 °C were set up. The specific procedures were as follows: 1) first, saturated solutions of a starting sample of the free base crystalline form A were prepared in corresponding solvents at corresponding temperatures; 2) the saturated solutions were filtered, and a proper amount of mixed crystal sample was weighed and added to each filtered saturated solution to form a suspension; 3) the suspensions were then stirred under corresponding temperature conditions. The results are summarized in Table 4-2, and the XRPD results are summarized in FIG. 2-4. The results indicate that, after competitive suspension in the n-heptane system at 50 °C and in the toluene system at room temperature/50 °C, the samples were all transformed into the free base crystalline form A, and after competitive suspension in the n-heptane system at room temperature for 42 days, the sample was transformed to the free base crystalline form A. The experiment shows that the free base crystalline form A is a thermodynamically stable crystalline form at room temperature and 50 °C.

**Table 4-2. Summary of competitive suspension tests**

| **Starting crystalline form** | **Solvent** | **Temperature** | **Time (d)** | **Results** |
|---|---|---|---|---|
| Free base crystalline forms A + B | n-Heptane | RT | 42 | Free base crystalline form A |
| | | 50 °C | 2 | Free base crystalline form A |
| | Toluene | RT | 5 | Free base crystalline form A |
| | | 50 °C | 2 | Free base crystalline form A |

### Example 5. Preparation and Screening of Salt Forms of Compound of Formula I

About 20 mg of the compound of formula I and equimolar amounts of different salt-forming formulations (i.e., acids for forming salts with the free base) were weighed into HPLC vials. Then, 0.5 mL of solvent was added to each vial for mixing to give a suspension. The salt-forming formulations were first diluted with the corresponding solvents before being mixed with the starting samples. The samples were suspended and stirred at room temperature for about 5 days, and then solids were separated out by centrifugation and dried under vacuum at room temperature overnight. For the room-temperature clarification systems, 0.5-1.0 mL of n-heptane as an antisolvent was added to increase the supersaturation of the solution for accelerating crystallization. The XRPD characterization results of the resulting solids show that a total of 5 salt forms were obtained in the salt form screening tests (Table 5-1).

**Table 5-1. Summary of the results of the salt form screening tests**

| No. | Acid ^{#} | A IPA/H₂O (19:1, v/v) | B EtOAc | C MTBE | D Acetone/ n-heptane (1:3, v/v) |
|---|---|---|---|---|---|
| 0 | Blank | Free base crystalline form A | Free base crystalline form A* | Free base crystalline form A | Free base crystalline form A |
| 1 | Hydrochloric acid | Free base crystalline form A+ Crystalline form A of hydrochloride | Crystalline form A of hydrochloride | Crystalline form A of hydrochloride | Crystalline form A of hydrochloride |
| 2 | Maleic acid | Free base crystalline form A | Crystalline form A of maleate* | Free base crystalline form A | Free base crystalline form A |
| 3 | L-aspartic acid | Free base crystalline form A+ L-aspartic acid | Aspartic acid | Free base crystalline form A+ L-aspartic acid | Free base crystalline form A+ L-aspartic acid |
| 4 | Phosphoric acid | Free base crystalline form A | Gelling* | Free base crystalline form A | Free base crystalline form A |
| 5 | Fumaric acid | Free base crystalline form A+ Fumaric acid | Fumaric acid | Free base crystalline form A+ Fumaric acid | Free base crystalline form A+ Fumaric acid |
| 6 | L-tartaric acid | Free base crystalline form A | Free base crystalline form A* | Free base crystalline form A | Free base crystalline form A |
| 7 | Citric acid | Free base crystalline form A+ multimodal | Free base crystalline form A* | Free base crystalline form A | Free base crystalline form A |
| 8 | D-glucuronic acid | Free base crystalline form A | Free base crystalline form A | Free base crystalline form A | Free base crystalline form A |
| 9 | L-malic acid | Free base crystalline form A+ | Free base crystalline form A* | Free base crystalline form A | Free base crystalline form A |
| | | multimodal | | | |
| 10 | Hippuric acid | Free base crystalline form A+ Hippuric acid | Hippuric acid | Free base crystalline form A+ Hippuric acid | Free base crystalline form A+ Hippuric acid |
| 11 12 | D-gluconic acid DL-lactic acid | Free base crystalline form A Free base crystalline form A | Free base crystalline form A* Free base crystalline form A* | Free base crystalline form A Free base crystalline form A | Free base crystalline form A Free base crystalline form A |
| 13 | Succinic acid | Free base crystalline form A | Free base crystalline form A+ Succinic acid* | Free base crystalline form A | Free base crystalline form A+ Succinic acid |
| 14 | L-ascorbic acid | Free base crystalline form A | Free base crystalline form A+ L-ascorbic acid | Free base crystalline form A+ L-ascorbic acid | Free base crystalline form A+ L-ascorbic acid |
| 15 | Adipic acid | Free base crystalline form A+ Adipic acid | Free base crystalline form A+ Adipic acid* | Free base crystalline form A+ Adipic acid | Free base crystalline form A+ Adipic acid |
| 16 | Acetic acid | Free base crystalline form A | Free base crystalline form A* | Free base crystalline form A | Free base crystalline form A |
| 17 | p-Toluenesulfoni c acid | Free base crystalline form A+ Crystalline form A of p-toluenesulfonate | Crystalline form A of p-toluenesulfonate | Crystalline form A of p-toluenesulfonate | D Crystalline form A of p-toluenesulfonate |
| 18 | Benzenesulfonic acid | Free base crystalline form A+ Crystalline form A of benzenesulfonate | Crystalline form A of benzene sulfonate | Crystalline form A of benzenesulfonate | Crystalline form A of benzenesulfonate |
| 19 | Oxalic acid | Free base crystalline form A | Free base crystalline form A* | Free base crystalline form A | Free base crystalline form A |
| 20 | 2-Hydroxyethane sulfonic acid | Free base crystalline form A | Gelling* | Gelling | Gelling |
| 21 | Malonic acid | Free base crystalline form A+ Crystalline form A of malonate | Crystalline form A of malonate* | Free base crystalline form A+ Crystalline form A of malonate | Crystalline form A of malonate |
| 22 | Gentisic acid | Free base crystalline form A | Free base crystalline form A* | Free base crystalline form A | Free base crystalline form A |
| 23 | Benzoic acid | Free base crystalline form A | Free base crystalline form A* | Free base crystalline form A | Free base crystalline form A |

| | | | | | |
|---|---|---|---|---|---|
| ^{#}: the feeding molar ratio of acid/base was 1:1. *: the suspension was stirred at room temperature for 3 h to give a clarified solution, and upon adding 0.5-1.0 mL of *n*-heptane, solid precipitation was observed. | | | | | |

### Example 6. Preparation of Crystalline Form A of Hydrochloride of Compound of Formula I

A sample of the compound of formula I and an equimolar amount of hydrochloric acid were slurried in MTBE at room temperature for 5 days, and then a solid was separated out by centrifugation and dried under vacuum at room temperature to give crystalline form A of hydrochloride.

The XRPD pattern of the sample of the crystalline form A of hydrochloride is shown in FIG. 3-1. ¹H NMR was measured in DMSO-*d₆,* and the results, as shown in FIG. 3-2, indicate that no solvent residue of MTBE was observed in the sample. The HPLC/IC results indicate that the hydrochloric acid and the API (i.e., free base) were in a molar ratio of 0.5:1.

The XRPD analysis data of the crystalline form A of hydrochloride of the compound of formula I obtained are shown in Table 6-1 below:

**Table 6-1. XRPD diffraction peak data of crystalline form A of hydrochloride**

| **Peak number** | **2θ [°]** | **Relative intensity [%]** |
|---|---|---|
| 1 | 7.7663 | 49.07 |
| 2 | 9.0069 | 50.47 |
| 3 | 10.0998 | 31.02 |
| 4 | 15.5379 | 100.00 |
| 5 | 17.5063 | 37.22 |
| 6 | 18.0142 | 19.02 |
| 7 | 19.2387 | 54.04 |
| 8 | 20.0478 | 16.17 |
| 9 | 21.2801 | 17.11 |
| 10 | 21.5911 | 12.54 |
| 11 | 22.6674 | 5.31 |
| 12 | 23.3757 | 15.53 |
| 13 | 23.7874 | 23.21 |
| 14 | 24.4926 | 58.16 |
| 15 | 26.0709 | 16.71 |
| 16 | 27.1737 | 13.31 |
| 17 | 28.3293 | 15.22 |
| 18 | 35.2229 | 3.84 |

### Example 7. Preparation of Crystalline Form A of Maleate of Compound of Formula I

A sample of the compound of formula I and an equimolar amount of maleic acid were stirred in EtOAc at room temperature for about 3 h, and 0.5 mL of n-heptane was added to the resulting clarified solution. The mixture was stirred at room temperature for 5 days, and then a solid was separated out by centrifugation and dried under vacuum at room temperature to give crystalline form A of maleate of the compound of formula I. The XRPD pattern of the sample of the crystalline form A of maleate is shown in FIG. 4-1. ¹H NMR was measured in DMSO-*d₆,* and the results are shown in FIG. 4-2. The results show that the maleic acid and the API (i.e., free base) were in a molar ratio of 0.5:1, and no solvent residues of EtOAc and n-heptane were observed.

The XRPD analysis data of the crystalline form A of maleate of the compound of formula I obtained are shown in Table 7-1 below:

**Table 7-1. XRPD diffraction peak data of crystalline form A of maleate**

| **Peak number** | **2θ [°]** | **Relative intensity [%]** |
|---|---|---|
| 1 | 5.4321 | 19.42 |
| 2 | 6.7257 | 41.00 |
| 3 | 9.9534 | 13.14 |
| 4 | 10.8368 | 62.03 |
| 5 | 11.7493 | 15.92 |
| 6 | 13.5038 | 5.30 |
| 7 | 14.6787 | 29.08 |
| 8 | 16.2629 | 32.26 |
| 9 | 16.8228 | 19.46 |
| 10 | 18.2265 | 47.35 |
| 11 | 18.4373 | 100.00 |
| 12 | 20.1675 | 12.97 |
| 13 | 22.7918 | 9.47 |
| 14 | 23.2244 | 8.74 |
| 15 | 24.0043 | 5.29 |
| 16 | 26.0738 | 7.60 |
| 17 | 27.7169 | 14.37 |
| 18 | 28.8575 | 4.11 |

### Example 8. Preparation of Crystalline Form A ofp-Toluenesulfonate of Compound of Formula I

A sample of the compound of formula I and an equimolar amount of *p*-toluenesulfonic acid were stirred in EtOAc at room temperature for 5 days, and then a solid was separated out by centrifugation and dried under vacuum at room temperature to give crystalline form A of *p*-toluenesulfonate of the compound of formula I. The XRPD pattern of the sample of the crystalline form A of *p*-toluenesulfonate is shown in FIG. 5-1. ¹H NMR was measured in DMSO-*d₆,* and the results are shown in FIG. 5-2. The results show that the p-toluenesulfonic acid and the API (i.e., free base) were in a molar ratio of 1.0:1, and no solvent residue of EtOAc was observed.

The XRPD analysis data of the crystalline form A of *p*-toluenesulfonate of the compound of formula I obtained are shown in Table 8-1 below:

**Table 8-1. XRPD diffraction peak data of crystalline form A ofp-toluenesulfonate**

| **Peak number** | **2θ [°]** | **Relative intensity [%]** |
|---|---|---|
| 1 | 4.9941 | 39.49 |
| 2 | 7.2577 | 75.74 |
| 3 | 8.6957 | 100.00 |
| 4 | 8.8744 | 44.39 |
| 5 | 14.4540 | 8.41 |
| 6 | 14.8759 | 9.94 |
| 7 | 15.1964 | 15.95 |
| 8 | 15.4025 | 46.78 |
| 9 | 16.4086 | 6.89 |
| 10 | 16.6812 | 17.13 |
| 11 | 17.4469 | 9.26 |
| 12 | 17.7319 | 31.06 |
| 13 | 19.1595 | 7.57 |
| 14 | 19.7137 | 14.34 |
| 15 | 20.6559 | 9.00 |
| 16 | 21.0073 | 36.67 |
| 17 | 21.7628 | 10.35 |
| 18 | 22.4064 | 4.05 |
| 19 | 24.1341 | 23.76 |
| 20 | 25.7570 | 8.40 |
| 21 | 26.1807 | 5.08 |
| 22 | 27.2494 | 11.37 |
| 23 | 27.9548 | 2.34 |
| 24 | 29.2297 | 2.06 |
| 25 | 30.6914 | 4.29 |
| 26 | 31.0030 | 4.48 |
| 27 | 31.7790 | 3.65 |
| 28 | 38.4148 | 1.51 |

### Example 9. Preparation of Crystalline Form A of Benzenesulfonate of Compound of Formula I

A sample of the compound of formula I and an equimolar amount of benzenesulfonic acid were stirred in MTBE at room temperature for 5 days, and then a solid was separated out by centrifugation and dried under vacuum at room temperature to give crystalline form A of benzenesulfonate of the compound of formula I. The XRPD pattern of the crystalline form A of benzenesulfonate is shown in FIG. 6-1. The TGA/DSC results are detailed in FIG. 6-2. The TGA results show that the sample had a weight loss of 1.35% when heated to 120 °C; the DSC results show that the sample had one endothermic peak with an onset temperature of 159.6 °C and a peak temperature of 160.9 °C. ¹H NMR was measured in DMSO-*d₆,* and the results are shown in FIG. 6-3. The results show that the benzenesulfonic acid and the API (i.e., free base) were in a molar ratio of 0.9:1, and no solvent residue of MTBE was observed.

The XRPD analysis data of the crystalline form A of benzene sulfonate of the compound of formula I obtained are shown in Table 9-1 below:

**Table 9-1. XRPD diffraction peak data of crystalline form A of benzenesulfonate**

| **Peak number** | **2θ [°]** | **Relative intensity [%]** |
|---|---|---|
| 1 | 5.3558 | 26.25 |
| 2 | 7.2756 | 83.67 |
| 3 | 8.3412 | 100.00 |
| 4 | 9.6394 | 42.23 |
| 5 | 10.6572 | 9.46 |
| 6 | 14.5488 | 4.68 |
| 7 | 14.9996 | 10.49 |
| 8 | 16.1998 | 64.25 |
| 9 | 16.9296 | 9.92 |
| 10 | 17.8527 | 10.59 |
| 11 | 18.5500 | 45.94 |
| 12 | 19.2801 | 16.96 |
| 13 | 19.7376 | 9.51 |
| 14 | 20.8009 | 9.33 |
| 15 | 21.4919 | 43.27 |
| 16 | 21.8091 | 23.06 |
| 17 | 23.2128 | 28.08 |
| 18 | 23.6766 | 9.48 |
| 19 | 23.9838 | 7.85 |
| 20 | 25.0545 | 17.37 |
| 21 | 25.7390 | 16.38 |
| 22 | 26.6532 | 12.85 |
| 23 | 27.8195 | 8.88 |
| 24 | 30.3976 | 2.24 |
| 25 | 37.3507 | 3.73 |

### Example 10. Preparation of Crystalline Form A of Malonate of Compound of Formula I

The compound of formula I and an equimolar amount of malonic acid were stirred in EtOAc at room temperature for about 3 h, and 0.5 mL of n-heptane was added to the resulting clarified solution. The mixture was stirred at room temperature for 5 days, and then a solid was separated out by centrifugation and dried under vacuum at room temperature to give crystalline form A of malonate of the compound of formula I. The XRPD pattern of the crystalline form A of malonate is shown in FIG. 7-1. The TGA/DSC results are detailed in FIG. 7-2. The TGA results show that the sample had a weight loss of 2.99% when heated to 120 °C, and had a weight loss of 11.02% when heated from 120 °C to 200 °C; the DSC results show that the sample had an endothermic peak at 156.4 °C (peak temperature). ¹H NMR was measured in DMSO-*d₆,* and the results are shown in FIG. 7-3. The results show that the malonic acid and the API (i.e., free base) were in a molar ratio of 0.7:1, and no solvent residue of EtOAc was observed.

The crystalline form A of malonate was subjected to crystalline form identification by VT-XRPD (FIG. 7-4), and the results indicate that no crystalline form changes were observed when the sample was purged under N₂ for 20 min, heated to 120 °C, and then cooled to room temperature.

The XRPD analysis data of the crystalline form A of malonate of the compound of formula I obtained are shown in Table 10-1 below:

**Table 10-1. XRPD diffraction peak data of crystalline form A of malonate**

| **Peak number** | **2θ [°]** | **Relative intensity [%]** |
|---|---|---|
| 1 | 5.3387 | 14.95 |
| 2 | 6.7490 | 50.46 |
| 3 | 9.9633 | 30.98 |
| 4 | 10.6723 | 45.47 |
| 5 | 11.8272 | 22.10 |
| 6 | 13.4912 | 20.74 |
| 7 | 14.4786 | 34.67 |
| 8 | 16.0367 | 32.39 |
| 9 | 16.8794 | 32.98 |
| 10 | 17.0411 | 24.12 |
| 11 | 18.0448 | 48.14 |
| 12 | 18.2855 | 100.00 |
| 13 | 18.6272 | 15.50 |
| 14 | 20.2750 | 17.74 |
| 15 | 21.5729 | 3.68 |
| 16 | 22.5685 | 10.00 |
| 17 | 22.9536 | 12.73 |
| 18 | 24.1081 | 5.50 |
| 19 | 24.8272 | 3.44 |
| 20 | 26.0170 | 2.91 |
| 21 | 27.3799 | 23.14 |
| 22 | 28.8258 | 13.77 |

### Example 11. Repeated Preparation of Polymorphs of Compound of Formula I

### (I) Repeated preparation of free base crystalline form B

**Table 11-1. Summary of repeat preparation results for free base crystalline form B**

| **Sample** | **Scale** | **Preparation method** | **Results** |
|---|---|---|---|
| 1 | 100 mg | The sample was completely dissolved in TFE, and then the solution was slowly volatilized at room temperature. | Free base crystalline form A |
| 2 | | | |
| 3 | 50 mg | The sample was completely dissolved in 1,4-dioxane, and then the solution was subjected to gas-liquid diffusion in an n-hexane atmosphere. | Free base crystalline forms A + B |
| 4 | | | Free base crystalline forms A + B |
| 5 | | | Free base crystalline forms A + B |
| 6 | | | Free base crystalline forms A + B |

As shown in Table 11-1, dissolving the free base crystalline form A in TFE did not yield the free base crystalline form B, so it remained as free base crystalline form A (the XRPD patterns of samples 1 and 2 are shown in FIG. 8-1); dissolving in 1,4-dioxane yield a mixed crystal of the free base crystalline forms A + B (the XRPD patterns of samples 3, 4, 5, and 6 are shown in FIG. 8-2).

### (II) Repeated preparation of salt forms

The crystalline form A of hydrochloride, the crystalline form A of maleate, and the crystalline form A of p-toluenesulfonate were selected for 300 mg repeated preparation. The results show that all three salt forms were successfully prepared repeatedly, and the procedures for repeated preparation of the salt form samples are summarized in Table 11-2.

**Table 11-2. Summary of procedures for repeated preparation of salt forms**

| **Salt form** | | **Preparation procedure** |
|---|---|---|
| Crystalline form A of hydrochloride | 1. | 301.2 mg of a sample of the compound of formula I was weighed into a 20 mL glass vial, and 7 mL of MTBE was added to give a suspension. |
| | 2. | 50 µL of 12 N HCl (at a feeding molar ratio of 1:1) was added. The mixture was stirred at room temperature for about 4 days, and then a solid was separated out and dried under vacuum at room temperature for about 3 h to give a sample (about 276 mg in total). |
| Crystalline form A of maleate | 1. | 300.6 mg of a sample of the compound of formula I and 70.5 mg of maleic acid (at a feeding molar ratio of 1:1) were weighed into a 20 mL glass vial. |
| | 2. | 5 mL of EtOAc was added, and the mixture was stirred at room temperature. After about 10 min, the sample was observed to be almost completely dissolved. |
| | 3. | Subsequently, 10 mL of n-heptane was added into the small vial, and then a large amount of solid precipitated. The mixture was stirred at room temperature for about 4 days, and then a solid was separated out and dried under vacuum at room temperature for about 3 h to give a sample (about 290 mg in total). |
| Crystalline form A of *p*-toluenesulfonate | 1. | 299.9 mg of a sample of the compound of formula I and 103.5 mg of *p*-toluenesulfonic acid (at a feeding molar ratio of 1:1) were weighed into a 20 mL glass vial, and 7 mL of EtOAc was added to give a suspension. |
| | 2. | The mixture was stirred at room temperature for about 4 days, and then a solid was separated out and dried under vacuum at room temperature for about 3 h to give a sample (about 321 mg in total). |

### 1. Crystalline form A of hydrochloride

The XRPD pattern of the sample of the crystalline form A of hydrochloride prepared repeatedly is shown in FIG. 9-1. The TGA/DSC results are shown in FIG. 9-2. The TGA results show that the sample had a weight loss of 2.2% when heated to 100 °C, and had a weight loss of 3.9% when heated from 100 °C to 160 °C; the DSC results show that the sample had endothermic peaks at 157.4 °C and 179.0 °C (peak temperature). ¹H NMR was measured in DMSO-*d₆*, and the results, as shown in FIG. 9-3, indicate that no MTBE solvent residue was observed. The HPLC/IC results indicate that the molar ratio of the sample of the crystalline form A of hydrochloride was 0.5:1 (hydrochloric acid:API). The PLM image (FIG. 9-5) shows that the crystalline form A of hydrochloride was in the form of small irregular particles. The crystalline form A of hydrochloride was subjected to crystalline form identification by VT-XRPD (FIG. 9-4), and the results indicate that no crystalline form changes were observed when the sample was purged under N₂ for 20 min, heated to 100 °C, and then cooled to room temperature.

### 2. Crystalline form A of maleate

The XRPD pattern of the sample of the crystalline form A of maleate prepared repeatedly is shown in FIG. 10-1. The TGA/DSC results are shown in FIG. 10-2. The TGA results show that the sample had a weight loss of 1.65% when heated to 110 °C, and had a weight loss of 11.88% when heated from 110 °C to 220 °C; the DSC results show that the sample had an endothermic peak at 144.1 °C (peak temperature). ¹H NMR was measured in DMSO-*d₆,* and the results are shown in FIG. 10-3. The results show that the maleic acid and the API in this sample were in a molar ratio of 0.5:1, and 0.3 wt% EtOAc solvent residue was observed. The PLM image (FIG. 10-5) shows that the crystalline form A of maleate was in the form of partially agglomerated irregular particles. The crystalline form A of maleate was subjected to crystalline form identification by VT-XRPD (FIG. 10-4), and the results indicate that no crystalline form changes were observed when the sample was purged under N₂ for 20 min, heated to 120 °C, and then cooled to room temperature.

### 3. Crystalline form A ofp-toluenesulfonate

The XRPD pattern of the sample of the crystalline form A of p-toluenesulfonate prepared repeatedly is shown in FIG. 11-1. The TGA/DSC results are shown in FIG. 11-2. The TGA results show that the sample had a weight loss of 0.73% when heated to 150 °C; the DSC results show that the sample had one endothermic peak with an onset temperature of 157.1 °C and a peak temperature of 159.2 °C. ¹H NMR was measured in DMSO-*d₆,* and the results are shown in FIG. 11-3. The results show that the p-toluenesulfonic acid and the API in this sample were in a molar ratio of 1.0:1, and no solvent residue of EtOAc was observed. The PLM image (FIG. 11-4) shows that the crystalline form A of p-toluenesulfonate was in the form of irregular particles.

### Example 12. Dynamic Solubility Experiment

Solids at a feeding concentration of 10 mg/mL (calculated based on free base) were rotationally mixed with corresponding vehicles at 37 °C. The solubility of each sample in four systems of water, SGF, FaSSIF and FeSSIF' was determined at different time points (1 h, 4 h, and 24 h). After sampling at each time point, the samples were centrifuged (at 10000 rpm) and filtered (through a 0.45 µm PTFE filter head). The HPLC concentrations and pH values of the filtrates were determined. The solubility test results are summarized in Table 12-1, and the solubility curves are shown in FIG. 12. The results indicate that the crystalline form A of maleate had the highest solubility in H₂O, and the crystalline form A of p-toluenesulfonate had the highest 1-hour solubility in the three biological vehicles other than H₂O. FIGs. 13-28 are XRPD overlays of the solubility samples of the free base crystalline form A, the crystalline form A of hydrochloride, the crystalline form A of maleate, and the crystalline form A of p-toluenesulfonate in H₂O, SGF, FaSSIF, and FeSSIF, respectively. The crystalline form changes are shown in Table 12-1. The free base crystalline form A did not undergo any crystalline form changes in the dynamic solubility tests, demonstrating very high stability.

**Table 12-1. Summary of dynamic solubility test results at 37 °C**

| **Starting material** | **Solvent** | **1 h** | | | **4h** | | | **24 h** | | |
|---|---|---|---|---|---|---|---|---|---|---|
| | | S | pH | Crystalline form change | S | pH | Crystalline form change | S | pH | Crystalline form change |
| Free base crystalline form A | H₂O | 0.003 | 8.8 | No | 0.003 | 8.9 | No | 0.003 | 9.0 | No |
| | SGF | 0.013 | 1.8 | No | 0.013 | 1.8 | No | 0.012 | 1.8 | No |
| | FaSSIF | 0.010 | 6.5 | No | 0.008 | 6.5 | No | 0.009 | 6.5 | No |
| | FeSSIF | 0.023 | 5.0 | No | 0.023 | 5.0 | No | 0.025 | 5.0 | No |
| Crystalline form A of hydrochloride | H₂O | 0.004 | 2.1 | Yes*^{a}* | 0.003 | 2.1 | Yes*^{a}* | 0.004 | 2.1 | Yes*^{a}* |
| | SGF | 0.014 | 1.6 | Yes*^{a}* | 0.011 | 1.6 | Yes*^{a}* | 0.011 | 1.6 | Yes*^{a}* |
| | FaSSIF | 0.009 | 5.2 | Yes*^{a}* | 0.009 | 5.2 | Yes*^{a}* | 0.014 | 5.2 | Yes*^{a}* |
| | FeSSIF | 0.030 | 4.9 | Yes*^{a}* | 0.027 | 4.9 | Yes*^{a}* | 0.023 | 4.9 | Yes*^{a}* |
| Crystalline form A of maleate | H₂O | 0.007 | 2.3 | Yes*^{a}* | 0.007 | 2.3 | Yes*^{a}* | 0.008 | 2.2 | Yes*^{a}* |
| | SGF | 0.010 | 1.7 | Yes*^{a}* | 0.010 | 1.7 | Yes*^{a}* | 0.009 | 1.7 | Yes*^{a}* |
| | FaSSIF | 0.006 | 4.9 | Yes*^{a}* | 0.007 | 4.7 | Yes*^{a}* | 0.007 | 4.5 | Yes*^{a}* |
| | FeSSIF | 0.020 | 4.9 | Yes*^{a}* | 0.020 | 4.9 | Yes*^{a}* | 0.021 | 4.9 | Yes*^{a}* |
| Crystalline form A of *p*-toluenesulfonate | H₂O | 0.006 | 1.8 | Yes*^{a}* | 0.005 | 1.8 | Yes*^{a}* | 1.8 | 0.005 | Yes*^{a}* |
| | SGF | 0.037 | 1.5 | Yes*^{b'}* | 0.033 | 1.5 | Yes*^{a+b'}* | 1.5 | 0.012 | Yes*^{a'}* |
| | FaSSIF | 0.028 | 2.6 | Yes*^{b'}* | 0.013 | 2.6 | Yes*^{a'}* | 2.6 | 0.006 | Yes*^{a'}* |
| | FeSSIF | 0.095 | 4.8 | Yes*^{b'}* | 0.081 | 4.8 | Yes*^{b}* | 4.8 | 0.028 | Yes*^{a'}* |

| | | | | | | | | | | |
|---|---|---|---|---|---|---|---|---|---|---|
| S: solubility (mg/mL); *^{a}*: transformed to free base crystalline form A; *^{b}*: transformed to free base crystalline form B; *^{a'}*: transformed to free base crystalline form A + additional diffraction peaks; *^{b'}*: transformed to free base crystalline form B + additional diffraction peaks; *^{a+b'}*: transformed to free base crystalline form A + free base crystalline form B + additional diffraction peaks. | | | | | | | | | | |

### Preparation description of biological vehicles:

### Preparation of simulated gastric fluid (SGF):

100.2 mg of NaCl and 50.3 mg of Triton X-100 were weighed into a 50 mL volumetric flask and then completely dissolved by addition of purified water. 816 µL of 1 M hydrochloric acid was added, and the pH was adjusted to 1.8 with 1 M hydrochloric acid or 1 M NaOH solution. The mixed solution was brought to the volume by addition of purified water.

### Preparation of fasted state simulated intestinal fluid (FaSSIF):

170.5 mg of anhydrous NaH₂PO₄, 22.2 mg of NaOH, and 310.9 mg of NaCl were weighed into a 50 mL volumetric flask and then completely dissolved by addition of purified water. The pH was adjusted to 6.5 with 1 M hydrochloric acid or 1 M NaOH solution. The mixed solution was brought to the volume by addition of purified water. Subsequently, 55.1 mg of SIF powder was weighed into a 25 mL volumetric flask and completely dissolved with the above solution, and the mixed solution was brought to the volume.

### Preparation of fed state simulated intestinal fluid (FeSSIF):

0.21 mL of glacial acetic acid, 101.6 mg of NaOH, and 295.4 mg of NaCl were added into a 25 mL volumetric flask and then completely dissolved by addition of about 20 mL of purified water. The pH was adjusted to 5.0 with 1 M hydrochloric acid or 1 M NaOH solution. The mixed solution was brought to the volume by addition of purified water, and then 280.8 mg of SIF powder was weighed into the flask and completely dissolved.

### Example 13. Hygroscopicity

Hygroscopicity of the free base crystalline form A prepared in Example 3, and the crystalline form A of hydrochloride, the crystalline form A of maleate and the crystalline form A of p-toluenesulfonate prepared repeatedly in Example 11 was evaluated using a dynamic vapor sorption (DVS) instrument. Starting at 0% RH, the test acquired the percentage changes in mass of the samples when the humidity changed (0% RH to 95% RH to 0% RH) at a constant temperature of 25 °C. The DVS test results and XRPD results for the samples before and after DVS test are shown in FIGs. 29-36. The results show that the vapor sorption of the free base crystalline form A at 25 °C/80% RH was about 0.91%, indicating that it has a slight hygroscopicity; the vapor sorption of the crystalline form A of hydrochloride at 25 °C/80% RH was about 0.49%, indicating that it has a slight hygroscopicity; the vapor sorption of the crystalline form A of maleate at 25 °C/80% RH was about 0.41%, indicating that it has a slight hygroscopicity, and when the humidity was more than 80% RH, rapid vapor sorption of the crystalline form A of maleate was observed; the vapor sorption of the sample of the crystalline form A of *p*-toluenesulfonate at 25 °C/80% RH was about 0.12%, indicating that it has almost no hygroscopicity. The crystalline forms of the samples of the free base crystalline form A, the crystalline form A of hydrochloride, the crystalline form A of maleate, and the crystalline form A of *p*-toluenesulfonate all remained unchanged after DVS test.

### Example 14. Solid State Stability

The free base crystalline form A prepared in Example 3, and the crystalline form A of hydrochloride, the crystalline form A of maleate and the crystalline form A of p-toluenesulfonate prepared repeatedly in Example 11 were placed in a sealed container at 60 °C for 1 day, and in an uncovered container at 25 °C/60% RH and 40 °C/75% RH for 1 week and 1 month (wherein the crystalline form A of hydrochloride and the crystalline form A of maleate were placed only for 1 week), and then the physical and chemical stability of the samples were tested by XRPD and HPLC. Purity data are shown in Table 14-1, and XRPD results are shown in FIGs. 37-40. The results show that the HPLC purity of the free base crystalline form A, the crystalline form A of hydrochloride, the crystalline form A of maleate, and the crystalline form A of *p*-toluenesulfonate showed no significant change after placement under the corresponding conditions.

**Table 14-1. Summary of solid state stability evaluation**

| **Crystalline form** | **Conditions** | | **HPLC results** | | |
|---|---|---|---|---|---|
| | | | Purity (area%) | Relative to starting (%) | Crystalline form |
| Free base crystalline form A | Starting | | 99.37 | -- | -- |
| | 60 °C/sealed | 1 day | 99.44 | 100.07 | |
| | 25 °C/60% RH/uncovered | 1 week | 99.38 | 100.01 | |
| | | 1 month | 99.33 | 99.96 | Free crystalline form A |
| | 40 °C/75% RH/uncovered | 1 week | 99.51 | 100.14 | |
| | | 1 month | 99.43 | 10006 | |
| Crystalline form A of hydrochloride | Starting | | 99.59 | -- | -- |
| | 60 °C/sealed | 1 day | 99.58 | 99.99 | Crystalline form A of hydrochloride |
| | 25 °C/60% RH/uncovered | 1 week | 99.57 | 99.98 | |
| | 40 °C/75% RH/uncovered | 1 week | 99.58 | 99.99 | |
| Crystalline form A of maleate | Starting | | 99.71 | -- | -- |
| | 60 °C/sealed | 1 day | 99.60 | 99.89 | |
| | 25 °C/60% RH/uncovered | 1 week | 99.71 | 100.00 | Crystalline form A of maleate |
| | 40 °C/75% RH/uncovered | 1 week | 99.65 | 99.94 | |
| Crystalline form A of *p*-toluenesulfonate | Starting | | 99.92 | -- | -- |
| | 60 °C/sealed | 1 day | 99.92 | 100.00 | |
| | 25 °C/60% RH/uncovered | 1 week | 99.92 | 100.00 | |
| | | 1 month | 99.92 | 100.00 | Crystalline form A of *p*-toluenesulfonate |
| | 40 °C/75% RH/uncovered | 1 week | 99.92 | 100.00 | |
| | | 1 month | 99.92 | 100.00 | |

Example 15. Different Preparation Processes for Free Base Crystalline Forms of Compound of Formula I The compound of formula I was prepared according to the procedures in Example 2, and free base crystalline form A or free base crystalline form B (identified by XRPD) was prepared according to the following methods.

### Method 1: Slow volatilization

A total of 6 slow volatilization tests were set up using different solvent systems. For each test, about 20 mg of a sample of the compound of formula I was weighed into a 5 mL vial, and 0.4-0.6 mL of the corresponding solvent in Table 15-1 was added. The sample was dissolved, and then the solution was filtered. The vial was sealed with a sealing film, and 2 pinholes were made on the film. The vial was then placed at room temperature for slow volatilization. The resulting solid was collected and subjected to an XRPD test. The test results, as shown in Table 15-1, indicate that free base crystalline forms A/B were obtained.

**Table 15-1. Summary of slow volatilization tests**

| **Solvent** | **Results** |
|---|---|
| TFE | Free base crystalline form B |
| Acetone | Free base crystalline form A |
| THF | Free base crystalline form A |
| DCM | Free base crystalline form A |
| ACN | Free base crystalline form A |
| EtOAc | Free base crystalline form A |

### Method 2: Gas-solid diffusion

A total of 8 gas-solid diffusion tests were set up using different solvents. For each test, about 20 mg of a sample of the compound of formula I was weighed into a 3 mL vial, and about 3 mL of a solvent was added into a 20 mL vial. The uncovered 3 mL vial was placed into the 20 mL vial, and then the 20 mL vial was sealed and allowed to stand at room temperature for 1-8 days. The solid was then collected and subjected to an XRPD test. The test results, as shown in Table 15-2, indicate that free base crystalline form A and free base crystalline forms A + B were obtained.

**Table 15-2. Summary of gas-solid diffusion tests**

| **Solvent** | **Results** |
|---|---|
| EtOH | Free base crystalline form A |
| DCM | Free base crystalline form A |
| ACN | Free base crystalline form A |
| THF | Free base crystalline forms A + B* |
| Acetone | Free base crystalline form A |
| Toluene | Free base crystalline form A |
| DMF | Free base crystalline form A |
| n-Hexane | Free base crystalline form A |

| | |
|---|---|
| *: obtained after volatilization at room temperature following clarification. | |

### Method 3: Gas-liquid diffusion

A total of 8 gas-liquid diffusion tests were set up using different solvents. For each test, about 20 mg of a sample of the compound of formula I was weighed into a 3 mL vial and then dissolved by addition of 0.4-2.2 mL of a solvent (the solution was filtered using a 0.45 µm PTFE filter head). About 3 mL of an antisolvent was added into a 20 mL vial. The uncovered 3 mL vial containing the clarified solution was placed into the 20 mL vial, and then the 20 mL vial was sealed and allowed to stand at room temperature. The resulting solid was collected and subjected to an XRPD test. The test results, as shown in Table 15-3, indicate that free base crystalline forms A/B and low crystallinity were obtained.

**Table 15-3. Summary of gas-liquid diffusion tests**

| **Solvent** | **Antisolvent** | **Results** |
|---|---|---|
| IPA | n-Pentane | Free base crystalline form A* |
| | Methyl butyl ether | Free base crystalline form A* |
| MIBK | Cyclohexane | Free base crystalline form B |
| | CPME | Free base crystalline form A* |
| 1,4-dioxane | Ether | Free base crystalline form A* |
| | n-Hexane | Free base crystalline form B |
| DMSO | H₂O | Free base crystalline form A |
| | m-Xylene | Low crystallinity* |

| | | |
|---|---|---|
| *: obtained after volatilization at room temperature. | | |

### Method 4: High polymer induction

A total of 8 high polymer induction tests were set up using 2 types of high polymer mixtures in different solvents. For each test, about 20 mg of a sample of the compound of formula I was weighed into a 3 mL vial and then dissolved by addition of 0.4-1.0 mL of a solvent. Then, the solution was filtered, and 2 mg of a polymer mixture was added. The vial was sealed with a sealing film, and 2 small holes were made on the film. The vial was then placed at room temperature for slow volatilization. The test results, as shown in Table 15-4, indicate that free base crystalline form A and free base crystalline forms A + B were obtained.

**Table 15-4. Summary of high polymer induction tests**

| **Solvent** | **High polymer** | **Results** |
|---|---|---|
| MeOH | A | Free base crystalline form A |
| MEK | | Free base crystalline form A |
| Methyl acetate | | Free base crystalline form A |
| THF | | Free base crystalline forms A + B |
| IPAc | B | Free base crystalline form A |
| EtOH | | Free base crystalline form A |
| DCM | | Free base crystalline form A |
| 2-MeTHF | | Free base crystalline form A |

| | | |
|---|---|---|
| High polymer mixture A: polyvinylpyrrolidone, polyvinyl alcohol, polyvinyl chloride, polyvinyl acetate, hydroxypropyl methylcellulose, and methylcellulose (mixed in equal mass) High polymer mixture B: polycaprolactone, polyethylene glycol, polymethylmethacrylate, sodium alginate, and hydroxyethylcellulose (mixed in equal mass) | | |

### Method 5: Humidity induction

A total of 5 humidity induction tests were set up using different solvents. For each test, about 20 mg of a sample of the compound of formula I was weighed into a 3 mL vial, and a saturated salt solution was prepared in a 20 mL vial at room temperature. The uncovered 3 mL vial was placed into the 20 mL vial, and then the 20 mL vial was sealed and allowed to stand at room temperature for 8 days. The solid was then collected and subjected to an XRPD test. The test results, as shown in Table 15-5, indicate that free base crystalline form A was obtained.

**Table 15-5. Summary of humidity induction tests**

| **Conditions** | **Results** |
|---|---|
| Saturated potassium acetate solution, ∼ 22.5% RH | Free base crystalline form A |
| Saturated potassium carbonate solution, ∼43.2% RH | Free base crystalline form A |
| Saturated sodium bromide solution, ∼57.6% RH | Free base crystalline form A |
| Saturated potassium bromide solution, ∼80.9% RH | Free base crystalline form A |
| H₂O, ∼100% RH | Free base crystalline form A |

### Method 6: Cyclic heating and cooling

A total of 9 cyclic heating and cooling tests were set up using different solvents. For each test, about 20 mg of a sample of the compound of formula I was weighed into an HPLC glass vial, and 0.5 mL of the corresponding solvent listed in Table 15-6 was added. The resulting suspension was magnetically stirred (1000 rpm) under temperature cycling (50-5 °C, 0.1 °C/min, 2 cycles), and then centrifuged (10000 rpm, 2 min). The solid was collected and subjected to an XRPD test. The test results, as shown in Table 15-6, indicate that free base crystalline form A was obtained.

**Table 15-6. Summary of cyclic heating and cooling tests**

| **Solvent (v/v)** | **Results** |
|---|---|
| n-Heptane | Free base crystalline form A |
| Methyl butyl ether | Free base crystalline form A |
| Anisole | Free base crystalline form A |
| Dicylohexylamine | Free base crystalline form A |
| Acetone/H₂O (1:4) | Free base crystalline form A |
| EtOH/H₂O (1:4) | Free base crystalline form A |
| EtOAc/Methylcyclohexane (1:9) | Free base crystalline form A |
| CHCl₃/n-Heptane (1:7) | Free base crystalline form A |
| MEK/m-Xylene(1:9) | Free base crystalline form A |

### Method 7. Antisolvent addition

A total of 12 antisolvent addition tests were set up using different solvents. For each test, about 20 mg of a sample of the compound of formula I was weighed into a 20 mL vial, and the solid was completely dissolved with 0.4-0.6 mL of a solvent (see Table 15-7). An antisolvent in Table 15-7 was added dropwise to the clarified solution while stirring (1000 rpm) until the solid precipitated; or after the total volume of the antisolvent was added to 5 mL, the sample without solid precipitation was suspended and stirred at 5 °C; if still no solid precipitated, the sample was suspended and stirred at -20 °C. The final clarified sample was volatilized at room temperature. The precipitated solid was separated out and subjected to an XRPD test. The results, as shown in Table 15-7, indicate that free base crystalline form A and a clarified solution were obtained in the antisolvent addition tests.

**Table 15-7. Summary of antisolvent addition tests**

| **Solvent** | **Antisolvent** | **Results** |
|---|---|---|
| MeOH | H₂O | Free base crystalline form A |
| | m-Xylene | Clarified |
| Acetone | H₂O | Free base crystalline form A |
| | n-Heptane | Free base crystalline form A |
| EtOAc | n-Pentane | Free base crystalline form A |
| | Cumene | Free base crystalline form A |
| THF | H₂O | Free base crystalline form A |
| DMAc | H₂O | Free base crystalline form A |
| NMP | H₂O | Free base crystalline form A |
| CHCl₃ | Cyclohexane | Free base crystalline form A |
| | p-Cymene | Free base crystalline form A |

| | | |
|---|---|---|
| *: after the antisolvent addition, a clarified solution was obtained, which was then stirred at 5 °C. | | |

### Method 8: Anti-antisolvent addition

A total of 8 anti-antisolvent addition tests were set up using different solvents. For each test, about 20 mg of a sample of the compound of formula I was weighed into a 20 mL vial, and the solid was completely dissolved with 0.4-0.6 mL of a solvent (see Table 15-8). The clarified solution was added dropwise to 5 mL of an antisolvent in Table 15-8 while stirring (1000 rpm); the sample without solid precipitation was suspended and stirred at 5 °C; if still no solid precipitated, the sample was suspended and stirred at -20 °C. The final clarified sample was volatilized at room temperature. The precipitated solid was separated out and subjected to an XRPD test. The results, as shown in Table 15-8, indicate that free base crystalline form A was obtained in the anti-antisolvent addition tests.

**Table 15-8. Summary of anti-antisolvent addition tests**

| **Antisolvent** | **Solvent** | **Results** |
|---|---|---|
| n-Heptane | EtOH | Free base crystalline form A |
| | EtOAc | Free base crystalline form A |
| | 2-MeTHF | Free base crystalline form A |
| Toluene | MEK | Free base crystalline form A^{#} |
| | ACN | Free base crystalline form A^{#} |
| | DCM | Free base crystalline form A* |
| H₂O | 1,4-dioxane | Free base crystalline form A |
| | EtOH | Free base crystalline form A |

| | | |
|---|---|---|
| *: the sample was obtained by stirring at 5 °C; : the sample was obtained by stirring at -20 °C. | | |

### Method 9. Room-temperature suspension stirring

A total of 15 room-temperature suspension stirring tests were set up using different solvents. For each test, about 20-40 mg of a sample of the compound of formula I was weighed into an HPLC glass vial, and 0.5 mL of the corresponding solvent listed in Table 15-9 was added. The resulting turbid solution was magnetically stirred (1000 rpm) at room temperature for about 4 days and then centrifuged (10000 rpm, 2 min). The solid was collected and subjected to an XRPD test. The test results, as shown in Table 15-9, indicate that free base crystalline form A was obtained.

**Table 15-9. Summary of room-temperature suspension stirring tests**

| **Solvent (v/v)** | **Results** |
|---|---|
| IBA | Free base crystalline form A |
| MTBE | Free base crystalline form A |
| Cyclohexane | Free base crystalline form A |
| Toluene | Free base crystalline form A |
| IPAc/Toluene (1:1) | Free base crystalline form A |
| H₂O | Free base crystalline form A |
| Methylcyclohexane | Free base crystalline form A |
| THF/n-Pentane (1:3) | Free base crystalline form A |
| Acetone/n-Pentane (1:4) | Free base crystalline form A |
| IPA/CPME (1:4) | Free base crystalline form A |
| MeOH/p-Cymene (1:4) | Free base crystalline form A |
| DCM/n-Heptane (1:5) | Free base crystalline form A |
| ACN/H₂O (1:1) | Free base crystalline form A |
| 1,4-dioxane/H₂O (1:1) | Free base crystalline form A |
| NMP/H₂O (1:3) | Free base crystalline form A |

### Method 10: Slow cooling

A total of 5 slow cooling tests were set up using different solvent systems. For each test, about 20 mg of a sample of the compound of formula I was weighed into an HPLC vial, and 1.0 mL of a solvent in Table 15-10 was added. The mixture was stirred at 50 °C for equilibration for about 2 h and then filtered (using a 0.45 µm PTFE filter head). The supernatant was collected, placed into a biological incubator, and cooled from 50 °C to 5 °C at 0.05 °C/min, and then the temperature was maintained at 5 °C. The clarified solution was transferred to -20 °C and the temperature was maintained. The precipitated solid was collected and subjected to an XRPD test, and the sample without solid precipitation was transferred to room temperature for volatilization. The test results, as shown in Table 15-10, indicate that free base crystalline form A and free base crystalline form B + multimodal were obtained in the slow cooling tests.

**Table 15-10. Summary of slow cooling tests**

| **Solvent (v/v)** | **Results** |
|---|---|
| IPA | Free base crystalline form A* |
| Anisole | Free base crystalline form A |
| IPAc | Free base crystalline form A* |
| MeOH/MTBE (1:4) | Free base crystalline form B + multimodal* |
| THF/H₂O (1:1) | Free base crystalline form A* |

| | |
|---|---|
| *: the sample was obtained by volatilization at room temperature. | |

### Method 11: 50 °C suspension stirring

A total of 16 suspension stirring tests at 50 °C are set up using different solvents. For each test, about 20-40 mg of a sample of the compound of formula I was weighed into an HPLC glass vial, and 0.5 mL of the corresponding solvent listed in Table 15-11 was added. The resulting suspension was magnetically stirred (1000 rpm) at 50 °C for about 3 days and then centrifuged (10000 rpm, 2 min). The solid was collected and subjected to an XRPD test. The test results, as shown in Table 15-11, indicate that free base crystalline form A was obtained.

**Table 15-11. Summary of 50 °C suspension stirring tests**

| **Solvent (v/v)** | **Results** |
|---|---|
| n-BuOH/Toluene (1:3) | Free base crystalline form A |
| Isopropyl ether | Free base crystalline form A |
| Methylcyclohexane | Free base crystalline form A |
| Cumene | Free base crystalline form A |
| Anisole | Free base crystalline form A |
| H₂O | Free base crystalline form A |
| PET | Free base crystalline form A |
| Dicyclohexylamine | Free base crystalline form A |
| 2 MeTHF/n-Hexane (1:5) | Free base crystalline form A |
| MEK/Cumene (1:5) | Free base crystalline form A |
| IPAc/Toluene (1:5) | Free base crystalline form A |
| CHCl₃/m-Xylene (1:4) | Free base crystalline form A |
| IPA/H₂O (1:2, v/v) | Free base crystalline form A |
| THF/H₂O (1:3, v/v) | Free base crystalline form A |
| MIBK/CPME (1:5) | Free base crystalline form A |
| Benzyl alcohol/Toluene (1:3) | Free base crystalline form A* |

| | |
|---|---|
| *: the sample was obtained after stirring at room temperature following clarification at 50 °C. | |

### Example 16.

### Synthesis of Control Compound 1 and Control Compound 2 with Reference to Patent Application No. WO 2016/091776A1

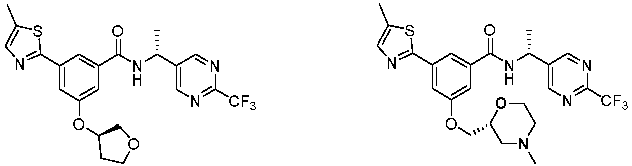

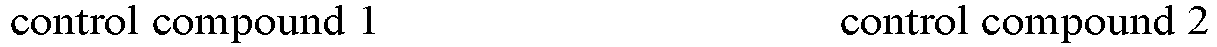

### I. Determination of antagonistic activity of hP2X3 antagonists against hP2X3 by FLIPR method

The antagonistic activity of human P2X3 receptor (hP2X3) antagonists against hP2X3 was evaluated using the FLIPR Calcium 4 Assay Kit (Molecular Devices, R8141) and FLIPR TETRA instrument (Molecular Devices, 0296) to detect calcium flux signals. 24 h before the experiment, human cells stably transfected with the hP2X3 receptor were seeded into a 384-well plate at a density of 2 × 10⁵ cells/mL, with 50 µL of cell suspension per well. The cells were then incubated in a 5% CO₂ incubator at 37 °C for 16-24 h. Each test compound was prepared in DMSO at 180 times the desired concentration (20-50 mM DMSO stock solution). 500 nL of the solution was then added to each well of the 384-well plate, followed by addition of 30 µL of FLIPR Assay buffer (1 × HBSS containing 1.26 mM Ca²⁺ + 2 mM CaCl₂, 20 mM HEPES). The mixture was shaken for 20-40 min to ensure homogeneous mixing. An agonist (α,β-meATP) was prepared using FLIPR Assay buffer at 3 times the desired concentration (desired final concentration of 400 nM). 45 µL of the agonist was then added to each well of another 384-well plate. The cell culture plate prepared one day ago was taken, and the cell supernatant was aspirated and discarded. 30 µL of Dye (FLIPR^{®} Calcium 4 Assay Kit, diluted in FLIPR buffer) was added to each well. The plate was then incubated for 1 h. 15 µL of the compound was added to the cells in each well (using the FLIPR instrument). After 15 min, 22.5 µL of the agonist was added to each well. The fluorescence signal was detected (with an excitation wavelength of 470-495 nm and an emission wavelength of 515-575 nm). Taking the difference between the peak and trough values of the signal as the base data, the data for the highest concentration of the positive drug as the 100% inhibition rate, and the DMSO data as the 0% inhibition rate, the inhibition effect curve of the compound was fitted on the software Graphpad Prism 6, and the IC₅₀ value was calculated.

**Table 16-1. Antagonistic activity of test compounds against hP2X3**

| Test compound | hP2X3 IC₅₀ (nM) |
|---|---|
| Control compound 1 | 241 |
| Compound of formula I | 67 |

### II. Pharmacokinetic assay in mice

Pharmacokinetic assay in mice was performed, wherein the mice used were male ICR mice weighing 20-25 g and fasted overnight. 3 mice were taken and given the compound by oral intragastric administration at 10 mg/kg, and blood was collected before administration and at 15 min, 30 min, 1 h, 2 h, 4 h, 8 h, and 24 h after administration. The blood samples were centrifuged at 6800 g at 2-8 °C for 6 min, and plasma was collected and stored at -80 °C. The plasma at each time point was taken, and 3-5 times its amount of an acetonitrile solution containing an internal standard was added for mixing. The mixture was vortexed for 1 min and then centrifuged at 13000 rpm at 4 °C for 10 min. The supernatant was taken, and 3 times its amount of water was added for mixing. A proper amount of the resulting mixture was taken for LC-MS/MS analysis. The major pharmacokinetic parameters were analyzed using a non-compartmental model in the WinNonlin 7.0 software.

The assay results indicate that in the mouse model, the compound of formula I of the present disclosure showed certain improvements in their pharmacokinetic properties compared to the control compounds 1 and 2.

**Table 16-2. Pharmacokinetics of test compounds in mice**

| Test compound | Pharmacokinetic parameters in mice (oral intragastric administration) | | | |
|---|---|---|---|---|
| | Cmax (ng/mL) | Tmax (hr) | AUC0-t (h*ng/mL) | T1/2 (hr) |
| Control compound 1 | 1246.6 | 0.50 | 5201.3 | 2.41 |
| Control compound 2 | 845.3 | 0.50 | 3186.1 | 2.66 |
| Compound of formula I | 3377.5 | 0.50 | 14974.5 | 2.89 |

### III. Taste assay in rats

After 3 days of overnight water-deprivation training, SD rats were subjected to administration with the compound. Half an hour after administration, each animal was given one bottle of water and one bottle of 0.3 mM aqueous quinine solution. The water bottles were removed after 15 min, and the intake of water and the intake of 0.3 mM aqueous quinine solution by the rats were measured separately. The effect of the compound on the taste of SD rats was assessed based on the difference between the water intake and the aqueous quinine solution intake (FIG. 41).

**Table 16-3. Water intake/aqueous quinine intake ratio for animals given the corresponding compounds**

| Compound | Water/ aqueous quinine solution |
|---|---|
| Vehicle | 34.7 |
| Control compound 1, 30 mg/kg, i.p. | 11.4 |
| Compound of formula I, 30 mg/kg, i.p. | 29.4 |

### IV. Cough efficacy assay of histamine/citric acid in guinea pigs

Before enrollment, the animals were acclimatized for 3-7 days. Once their body weight reached the standard (300-400 g), they were numbered and randomly grouped.

0.25-24 h before the start of the cough assessment, the compound or excipient was administered to the guinea pigs via intranasal instillation. The dose range for the administration of the test substance was 0.17-1.5 mg/kg. During the cough assessment, after the animals were acclimated in a whole-body plethysmography box, they were given histamine nebulization, followed by citric acid nebulization. The number of coughs and the cough latency of the animals were recorded within 22 min from the start of the histamine nebulization until the end of the observation period.

For the statistics of experimental data, the one-way ANOVA method was used to analyze and compare the data of the groups. Statistical analysis results with p < 0.05 were considered to indicate significant differences. Pairwise comparisons were performed using the t-test method to assess differences.

**Table 16-4. The number of coughs stimulated by histamine/citric acid in guinea pigs after administration of corresponding compounds**

| **Compound** | **Mean number of coughs within 15 min** | **Cough inhibition rate vs. vehicle (%)** |
|---|---|---|
| Vehicle | 26.6 | -- |
| Control compound 1 | 11.4 | 57.1 |
| Compound of formula I | 8.9 | 66.5 |

The data show that compared to the control compound 1, the compound of the present disclosure significantly reduced the number of coughs in guinea pigs in the citric acid/histamine-stimulated cough model, prolonged the cough latency, and exhibited a good antitussive effect (FIG. 42).

### V. Cough efficacy assay of ATP/citric acid in guinea pigs

Before enrollment, the animals were acclimatized for 3-7 days. Once their body weight reached the standard (300-400 g), they were numbered and randomly grouped. 0.25-24 h before the start of the cough assessment, the compound or excipient was administered to the guinea pigs via intranasal instillation. The dose range for the administration of the test substance was 0.17-1.5 mg/kg. During the cough assessment, after the animals were acclimated in a whole-body plethysmography box, they were given ATP nebulization, and after a few minutes, they were given citric acid nebulization. The number of coughs and the cough latency of the animals were recorded within 15 min from the start of the citric acid nebulization.

The one-way ANOVA method was used to analyze and compare the data of the groups. Statistical analysis results with p < 0.05 were considered to indicate significant differences. Pairwise comparisons were performed using the t-test method to assess differences.

**Table 16-5. The number of coughs stimulated by ATP/citric acid in guinea pigs after administration of corresponding compounds**

| **Compound** | **Mean number of coughs within 15 min** | **Cough inhibition rate vs. vehicle (%)** |
|---|---|---|
| Vehicle | 19.8 | -- |
| Control compound 1 | 8.1 | 59.1 |
| Compound of formula I | 6.3 | 68.2 |

The data show that compared to the control compound 1, the compound of the present disclosure significantly reduced the number of coughs in guinea pigs in the citric acid//ATP-stimulated cough model, prolonged the cough latency, and exhibited a good antitussive effect (FIG. 43).

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. A crystalline form of a compound of formula I or a pharmaceutically acceptable salt thereof, wherein the compound of formula I has a structure shown as follows:

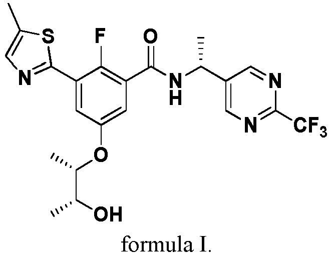

2. The crystalline form as claimed in claim 1, wherein the crystalline form is free base crystalline form A of the compound of formula I, and the free base crystalline form A has an X-ray powder diffraction pattern comprising diffraction peaks at 2θ±0.2° diffraction angles of 7.44°, 14.87°, 15.77°, 17.81°, and 18.61°;
preferably, the free base crystalline form A has an X-ray powder diffraction pattern comprising diffraction peaks at 2θ±0.2° diffraction angles of 7.44°, 11.14°, 11.36°, 14.87°, 15.77°, 16.97°, 17.81°, and 18.61°;
preferably, the free base crystalline form A has an X-ray powder diffraction pattern comprising diffraction peaks at 2Θ±0.2° diffraction angles of 3.75°, 7.44°, 11.14°, 11.36°, 11.98°, 12.25°, 14.87°, 15.77°, 16.97°, 17.81°, 18.61°, and 22.36°;
preferably, the free base crystalline form A has an X-ray powder diffraction pattern comprising diffraction peaks at 2Θ±0.2° diffraction angles of 3.75°, 5.99°, 7.44°, 9.01°, 9.93°, 11.14°, 11.36°, 11.98°, 12.25°, 13.88°, 14.20°, 14.87°, 15.77°, 16.97°, 17.81°, 18.61°, 22.36°, and 24.07°;
preferably, the free base crystalline form A has an X-ray powder diffraction pattern comprising diffraction peaks at 2Θ±0.2° diffraction angles of 3.75°, 5.99°, 7.44°, 9.01°, 9.93°, 11.14°, 11.36°, 11.98°, 12.25°, 13.88°, 14.20°, 14.87°, 15.77°, 16.97°, 17.81°, 18.61°, 19.39°, 20.26°, 21.14°, 22.36°, 23.34°, 24.07°, 26.33°, 26.78°, 27.18°, 28.17°, 30.20°, 33.88°, 34.35°, 37.23°, and 37.70°;
preferably, the free base crystalline form A has an XRPD pattern substantially as shown in FIG. 1-1;
preferably, the free base crystalline form A has one, two, or three of the following characteristics:
(1) a TGA curve of the free base crystalline form A showing a weight loss of about 1.28±1% at 150.0±3 °C;
(2) a DSC curve of the free base crystalline form A having a starting point of an endothermic peak at 175.6±3 °C; and
(3) the DSC curve of the free base crystalline form A having an endothermic peak at 176.4±3 °C;
preferably, a TGA/DSC profile of the free base crystalline form A is shown in FIG. 1-2;
preferably, a ¹H NMR spectrum of the free base crystalline form A is shown in FIG. 1-3.

3. The crystalline form as claimed in claim 1, wherein the crystalline form is free base crystalline form B of the compound of formula I, wherein the free base crystalline form B has an X-ray powder diffraction pattern comprising diffraction peaks at 2Θ±0.2° diffraction angles of 7.21°, 12.48°, 13.17°, 14.41°, 19.09°, 19.56°, 22.09°, and 26.49°;
preferably, the free base crystalline form B has an X-ray powder diffraction pattern comprising diffraction peaks at 2Θ±0.2° diffraction angles of 7.21°, 12.48°, 13.17°, 14.41°, 16.72°, 19.09°, 19.56°, 20.90°, 22.09°, and 26.49°;
preferably, the free base crystalline form B has an X-ray powder diffraction pattern comprising diffraction peaks at 2Θ±0.2° diffraction angles of 7.21°, 8.35°, 12.48°, 13.17°, 14.41°, 15.05°, 16.72°, 17.80°, 18.39°, 19.09°, 19.56°, 20.90°, 21.67°, 22.09°, 22.97°, 25.16°, 26.49°, and 27.49°;
preferably, the free base crystalline form B has an X-ray powder diffraction pattern comprising diffraction peaks at 2Θ±0.2° diffraction angles of 7.21°, 8.35°, 12.48°, 13.17°, 14.41°, 15.05°, 16.72°, 17.80°, 18.39°, 19.09°, 19.56°, 20.90°, 21.67°, 22.09°, 22.97°, 25.16°, 25.45°, 26.49°, 27.49°, 28.66°, 29.10°, 29.35°, 31.71°, 32.00°, 32.85°, 33.70°, 34.23°, 36.78°, 38.26°, and 38.70°;
preferably, the free base crystalline form B has an XRPD pattern substantially as shown in FIG. 2-1;
preferably, the free base crystalline form B has one, two, or three of the following characteristics:
(1) a TGA curve of the free base crystalline form B showing a weight loss of about 2.36±1% at 150.0±3 °C;
(2) a DSC curve of the free base crystalline form B having a starting point of an endothermic peak at 177.0±3 °C; and
(3) the DSC curve of the free base crystalline form B having an endothermic peak at 179.4±3 °C.

4. The crystalline form as claimed in claim 1, wherein the pharmaceutically acceptable salt of the compound of formula I comprises a salt formed by the compound of formula I with an inorganic acid or an organic acid; the inorganic acid comprises: hydrochloric acid, sulfuric acid, phosphoric acid, nitric acid, and hydrobromic acid;
the organic acid comprises: maleic acid, L-aspartic acid, fumaric acid, L-tartaric acid, citric acid, D-glucuronic acid, L-malic acid, hippuric acid, D-gluconic acid, DL-lactic acid, succinic acid, L-ascorbic acid, adipic acid, acetic acid, p-toluenesulfonic acid, methanesulfonic acid, benzenesulfonic acid, oxalic acid, 2-hydroxyethanesulfonic acid, malonic acid, gentisic acid, and benzoic acid;
preferably, the pharmaceutically acceptable salt of the compound of formula I is a hydrochloride, a maleate, a *p*-toluenesulfonate, a benzene sulfonate, or a malonate of the compound of formula I.

5. The crystalline form as claimed in claim 1, wherein the crystalline form of the pharmaceutically acceptable salt of the compound of formula I is crystalline form A of hydrochloride, crystalline form A of maleate, crystalline form A of *p*-toluenesulfonate, crystalline form A of benzenesulfonate, or crystalline form A of malonate of the compound of formula I.

6. The crystalline form as claimed in claim 5, wherein the crystalline form A of hydrochloride has an X-ray powder diffraction pattern comprising diffraction peaks at 2θ=0.2° diffraction angles of 7.77°, 9.01°, 10.10°, 15.54°, 17.51°, 19.24°, and 24.49°;
preferably, the crystalline form A of hydrochloride has an X-ray powder diffraction pattern comprising diffraction peaks at 2θ±0.2° diffraction angles of 7.77°, 9.01°, 10.10°, 15.54°, 17.51°, 18.01°, 19.24°, 20.05°, 21.28°, 23.38°, 23.79°, 24.49°, 26.07°, and 28.33°;
preferably, the crystalline form A of hydrochloride has an X-ray powder diffraction pattern comprising diffraction peaks at 2θ±0.2° diffraction angles of 7.77°, 9.01°, 10.10°, 15.54°, 17.51°, 18.01°, 19.24°, 20.05°, 21.28°, 21.59°, 22.67°, 23.38°, 23.79°, 24.49°, 26.07°, 27.17°, and 28.33°;
preferably, the crystalline form A of hydrochloride has an XRPD pattern substantially as shown in FIG. 3-1;
preferably, in the crystalline form A of hydrochloride, the compound of formula I and the hydrochloric acid are in a molar ratio of 2:1;
preferably, the crystalline form A of hydrochloride has a VT-XRPD pattern substantially as shown in FIG. 9-4;
preferably, the crystalline form A of hydrochloride has one, two, three, four, five, or six of the following characteristics:
(1) a TGA curve of the crystalline form A of hydrochloride showing a weight loss of about 2.19±1% at 100.0±3 °C;
(2) the TGA curve of the crystalline form A of hydrochloride showing a weight loss of about 3.90±1% in a temperature range of 100.0±3 °C to 160.0±3 °C;
(3) a DSC curve of the crystalline form A of hydrochloride having a starting point of an endothermic peak at 143.6±3 °C;
(4) the DSC curve of the crystalline form A of hydrochloride having an endothermic peak at 157.4±3 °C;
(5) the DSC curve of the crystalline form A of hydrochloride having an endothermic peak at 176.0±3 °C; and
(6) the DSC curve of the crystalline form A of hydrochloride having an endothermic peak at 179.0±3 °C.

7. The crystalline form as claimed in claim 5, wherein the crystalline form A of maleate has an X-ray powder diffraction pattern comprising diffraction peaks at 2θ±0.2° diffraction angles of 6.73°, 10.84°, 14.68°, 16.26°, 18.23°, and 18.44°;
preferably, the crystalline form A of maleate has an X-ray powder diffraction pattern comprising diffraction peaks at 2θ±0.2° diffraction angles of 5.43°, 6.73°, 10.84°, 14.68°, 16.26°, 16.82°, 18.23°, and 18.44°;
preferably, the crystalline form A of maleate has an X-ray powder diffraction pattern comprising diffraction peaks at 2θ±0.2° diffraction angles of 5.43°, 6.73°, 9.95°, 10.84°, 11.75°, 13.50°, 14.68°, 16.26°, 16.82°, 18.23°, 18.44°, 20.17°, 22.79°, 23.22°, 24.00°, 26.07°, 27.72°, and 28.86°;
preferably, the crystalline form A of maleate has an XRPD pattern substantially as shown in FIG. 4-1;
preferably, in the crystalline form A of maleate, the compound of formula I and the maleic acid are in a molar ratio of 2:1;
preferably, the crystalline form A of maleate has a VT-XRPD pattern substantially as shown in FIG. 10-4;
preferably, the crystalline form A of maleate has one, two, three, or more of the following characteristics:
(1) a TGA curve of the crystalline form A of maleate showing a weight loss of about 1.65±1% at 110.0±3 °C;
(2) the TGA curve of the crystalline form A of maleate showing a weight loss of about 11.88±1% in a temperature range of 110.0±3 °C to 220.0±3 °C;
(3) a DSC curve of the crystalline form A of maleate having an endothermic peak at 107.8±3 °C;
(4) the DSC curve of the crystalline form A of maleate having a starting point of an endothermic peak at 143.4±3 °C;
(5) the DSC curve of the crystalline form A of maleate having an endothermic peak at 144.1±3 °C; and
(6) the DSC curve of the crystalline form A of maleate having an endothermic peak at 160.2±3 °C.

8. The crystalline form as claimed in claim 5, wherein the crystalline form A of p-toluenesulfonate has an X-ray powder diffraction pattern comprising diffraction peaks at 2θ±0.2° diffraction angles of 4.99°, 7.26°, 8.70°, 8.87°, 15.40°, 17.73°, 21.01°, and 24.13°;
preferably, the crystalline form A of *p*-toluenesulfonate has an X-ray powder diffraction pattern comprising diffraction peaks at 2θ±0.2° diffraction angles of 4.99°, 7.26°, 8.70°, 8.87°, 15.20°, 15.40°, 16.68°, 17.73°, 19.71°, 21.01°, and 24.13°;
preferably, the crystalline form A of *p*-toluenesulfonate has an X-ray powder diffraction pattern comprising diffraction peaks at 2θ±0.2° diffraction angles of 4.99°, 7.26°, 8.70°, 8.87°, 14.45°, 14.88°, 15.20°, 15.40°, 16.41°, 16.68°, 17.45°, 17.73°, 19.16°, 19.71°, 20.66°, 21.01°, 21.76°, 22.41°, 24.13°, 25.76°, 26.18°, and 27.25°;
preferably, the crystalline form A of *p*-toluenesulfonate has an X-ray powder diffraction pattern comprising diffraction peaks at 2θ±0.2° diffraction angles of 4.99°, 7.26°, 8.70°, 8.87°, 14.45°, 14.88°, 15.20°, 15.40°, 16.41°, 16.68°, 17.45°, 17.73°, 19.16°, 19.71°, 20.66°, 21.01°, 21.76°, 22.41°, 24.13°, 25.76°, 26.18°, 27.25°, 27.95°, 29.23°, 30.69°, 31.00°, 31.78°, and 38.41°;
preferably, the crystalline form A of *p*-toluenesulfonate has an XRPD pattern substantially as shown in FIG. 5-1;
preferably, in the crystalline form A of p-toluenesulfonate, the compound of formula I and the p-toluenesulfonic acid are in a molar ratio of 1:1;
preferably, the crystalline form A of p-toluenesulfonate has one, two, or three of the following characteristics:
(1) a TGA curve of the crystalline form A of *p*-toluenesulfonate showing a weight loss of about 0.73±1% at 150.0±3 °C;
(2) a DSC curve of the crystalline form A of *p*-toluenesulfonate having a starting point of an endothermic peak at 157.1±3 °C; and
(3) the DSC curve of the crystalline form A of *p*-toluenesulfonate having an endothermic peak at 159.2±3 °C.

9. The crystalline form as claimed in claim 5, wherein the crystalline form A of benzene sulfonate has an X-ray powder diffraction pattern comprising diffraction peaks at 2θ±0.2° diffraction angles of 5.36°, 7.28°, 8.34°, 9.64°, 16.20°, 18.55°, and 21.49°;
preferably, the crystalline form A of benzenesulfonate has an X-ray powder diffraction pattern comprising diffraction peaks at 2θ±0.2° diffraction angles of 5.36°, 7.28°, 8.34°, 9.64°, 16.20°, 18.55°, 19.28°, 21.49°, 21.81°, 23.21°, 25.05°, and 25.74°;
preferably, the crystalline form A of benzenesulfonate has an X-ray powder diffraction pattern comprising diffraction peaks at 2θ±0.2° diffraction angles of 5.36°, 7.28°, 8.34°, 9.64°, 10.66°, 14.55°, 15.00°, 16.20°, 16.93°, 17.85°, 18.55°, 19.28°, 19.74°, 20.80°, 21.49°, 21.81°, 23.21°, 23.68°, 23.98°, 25.05°, 25.74°, 26.65°, and 27.82°;
preferably, the crystalline form A of benzene sulfonate has an XRPD pattern substantially as shown in FIG. 6-1;
preferably, in the crystalline form A of benzenesulfonate, the compound of formula I and the benzenesulfonic acid are in a molar ratio of 1:1;
preferably, the crystalline form A of benzenesulfonate has one, two, or three of the following characteristics:
(1) a TGA curve of the crystalline form A of benzene sulfonate showing a weight loss of about 1.35±1% at 120.0±3 °C;
(2) a DSC curve of the crystalline form A of benzene sulfonate having a starting point of an endothermic peak at 159.6±3 °C; and
(3) the DSC curve of the crystalline form A of benzenesulfonate having an endothermic peak at 160.9±3 °C.

10. The crystalline form as claimed in claim 5, wherein the crystalline form A of malonate has an X-ray powder diffraction pattern comprising diffraction peaks at 2θ=0.2° diffraction angles of 6.75°, 9.96°, 10.67°, 14.48°, 16.04°, 16.88°, 18.04°, and 18.29°;
preferably, the crystalline form A of malonate has an X-ray powder diffraction pattern comprising diffraction peaks at 2θ±0.2° diffraction angles of 5.34°, 6.75°, 9.96°, 10.67°, 11.83°, 13.49°, 14.48°, 16.04°, 16.88°, 17.04°, 18.04°, 18.29°, and 27.38°;
preferably, the crystalline form A of malonate has an X-ray powder diffraction pattern comprising diffraction peaks at 2θ±0.2° diffraction angles of 5.34°, 6.75°, 9.96°, 10.67°, 11.83°, 13.49°, 14.48°, 16.04°, 16.88°, 17.04°, 18.04°, 18.29°, 20.27°, 22.57°, 22.95°, 27.38°, and 28.83°;
preferably, the crystalline form A of malonate has an X-ray powder diffraction pattern comprising diffraction peaks at 2θ±0.2° diffraction angles of 5.34°, 6.75°, 9.96°, 10.67°, 11.83°, 13.49°, 14.48°, 16.04°, 16.88°, 17.04°, 18.04°, 18.29°, 18.63°, 20.27°, 21.57°, 22.57°, 22.95°, 24.11°, 24.83°, 26.02°, 27.38°, and 28.83°;
preferably, the crystalline form A of malonate has an XRPD pattern substantially as shown in FIG. 7-1;
preferably, in the crystalline form A of malonate, the compound of formula I and the malonic acid are in a molar ratio of 2:1;
preferably, the crystalline form A of malonate has a VT-XRPD pattern substantially as shown in FIG. 7-4;
preferably, the crystalline form A of malonate has one, two, three, four, or five of the following characteristics:
(1) a TGA curve of the crystalline form A of malonate showing a weight loss of about 2.99±1% at 120.0±3 °C;
(2) the TGA curve of the crystalline form A of malonate showing a weight loss of about 11.02±1% in a temperature range of 120.0±3 °C to 200.0±3 °C;
(3) a DSC curve of the crystalline form A of malonate having a starting point of an endothermic peak at 155.6±3 °C;
(4) the DSC curve of the crystalline form A of malonate having an endothermic peak at 156.4±3 °C; and
(5) the DSC curve of the crystalline form A of malonate having an endothermic peak at 172.4±3 °C.

11. A salt of a compound of formula I, wherein a pharmaceutically acceptable salt of the compound of formula I comprises a salt formed by the compound of formula I with an inorganic acid or an organic acid; the compound of formula I has a structure shown as follows: 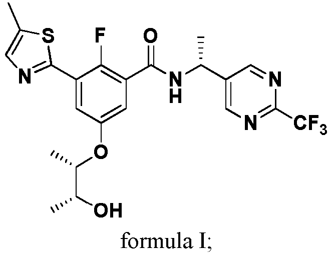
the inorganic acid comprises: hydrochloric acid, sulfuric acid, phosphoric acid, nitric acid, and hydrobromic acid;
the organic acid comprises: maleic acid, L-aspartic acid, fumaric acid, L-tartaric acid, citric acid, D-glucuronic acid, L-malic acid, hippuric acid, D-gluconic acid, DL-lactic acid, succinic acid, L-ascorbic acid, adipic acid, acetic acid, *p*-toluenesulfonic acid, methanesulfonic acid, benzenesulfonic acid, oxalic acid, 2-hydroxyethanesulfonic acid, malonic acid, gentisic acid, and benzoic acid;
preferably, the pharmaceutically acceptable salt of the compound of formula I is a hydrochloride, a maleate, a p-toluenesulfonate, a benzenesulfonate, or a malonate of the compound of formula I;
preferably, the compound of formula I and the acid are in a molar ratio of 5:1 to 1:5, such as 3:1, 2:1, 1:1, 1:1.5, 1:2, 1:2.5, or 1:3. preferably, the compound of formula I and the acid are in a molar ratio of 1:1 or 2:1.

12. A preparation method for the free base crystalline form A of the compound of formula I as claimed in claim 2, being selected from any one of the following methods:
Method 1, comprising adding the compound of formula I to an organic solvent I for dissolution, filtering, and volatilizing at room temperature,
wherein preferably, the organic solvent I is selected from one or more of acetone, tetrahydrofuran, dichloromethane, acetonitrile, and ethyl acetate;
Method 2, comprising completely dissolving the compound of formula I in an organic solvent II, and dropwise adding an antisolvent to the clarified solution under stirring until a solid precipitates, wherein if no solid precipitates, suspension stirring is adopted; if still no solid precipitates, suspension stirring is performed at a reduced temperature, and then the clarified solution is volatilized at room temperature;
the organic solvent II is selected from one or more of methanol, acetone, ethyl acetate, tetrahydrofuran, trichloromethane, *N,N*-dimethylacetamide, and *N*-methylpyrrolidone;
the antisolvent is selected from one or more of water, *m*-xylene, *n*-hexane, cumene, toluene, cyclohexane, *n*-heptane, *n*-pentane, and *p*-cymene;
Method 3, comprising placing an uncovered first sample bottle containing the compound of formula I into a second sample bottle containing a solvent, sealing the second sample bottle, and allowing the second sample bottle to stand at room temperature, wherein the solvent does not cover a mouth of the first sample bottle;
the solvent is selected from one or more of ethanol, dichloromethane, acetonitrile, acetone, toluene, *N,N*-dimethylacetamide, and *n*-hexane;
Method 4, comprising placing an uncovered first sample bottle containing a solution of the compound of formula I into a second sample bottle containing an antisolvent, sealing the second sample bottle, and allowing the second sample bottle to stand at room temperature, wherein the antisolvent does not cover a mouth of the first sample bottle;
the solvent in the solution of the compound of formula I is selected from one or more of isopropanol, methyl isobutyl ketone, 1,4-dioxane, and dimethylsulfoxide;
the antisolvent is selected from one or more of n-pentane, methyl butyl ether, water, and m-xylene;
Method 5, comprising adding a polymer to a solution of the compound of formula I and allowing the mixture to volatilize at room temperature,
wherein the solvent in the solution of the compound of formula I is selected from one or more of methanol, 2-butanone, methyl acetate, isopropyl acetate, ethanol, dichloromethane, and 2-methyltetrahydrofuran;
the polymer is selected from one or more of polyvinylpyrrolidone, polyvinyl alcohol, polyvinyl chloride, polyvinyl acetate, hydroxypropyl methylcellulose, methylcellulose, polycaprolactone, polyethylene glycol, polymethylmethacrylate, sodium alginate, and hydroxyethylcellulose;
Method 6, comprising placing an uncovered first sample bottle containing the compound of formula I into a second sample bottle containing a saturated salt solution or water, sealing the second sample bottle, and allowing the second sample bottle to stand at room temperature, wherein the solvent does not cover a mouth of the first sample bottle;
preferably, the saturated salt solution is a saturated inorganic salt solution;
preferably, the saturated inorganic salt solution is a saturated potassium acetate solution, a saturated potassium carbonate solution, a saturated sodium bromide solution, or a saturated potassium bromide solution;
preferably, the humidity of the system is 15%-100% RH;
Method 7, comprising stirring a suspension of the compound of formula I under temperature cycling, performing centrifugation, and collecting a solid,
wherein a solvent in the suspension is selected from one or more of n-heptane, methyl butyl ether, anisole, dicyclohexylamine, acetone, ethanol, ethyl acetate, methylcyclohexane, trichloromethane, 2-butanone, m-xylene, and water;
preferably, conditions for the temperature cycling comprise: 50 °C to 5 °C, 0.1-0.5 °C/min, and at least 2 cycles;
Method 8, comprising completely dissolving the compound of formula I in a good solvent, and dropwise adding an antisolvent to the clarified solution under stirring until a solid precipitates, wherein if no solid precipitates, suspension stirring is adopted; if still no solid precipitates, suspension stirring is performed at a reduced temperature, and then the clarified solution is volatilized at room temperature;
the good solvent is selected from one or more of ethanol, ethyl acetate, 2-methyltetrahydrofuran, 2-butanone, acetonitrile, dichloromethane, and 1,4-dioxane;
the antisolvent is selected from one or more of n-heptane, tetrahydrofuran, and water;
Method 9, comprising placing a turbid solution of the compound of formula I at room temperature with magnetic stirring, performing centrifugation, and collecting a solid,
wherein a solvent in the turbid solution is selected from one or more of isobutanol, methyl tert-butyl ether, cyclohexane, toluene, isopropyl acetate, water, methylcyclohexane, tetrahydrofuran, n-pentane, acetone, isopropanol, cyclopentyl methyl ether, methanol, p-cymene, dichloromethane, n-heptane, acetonitrile, 1,4-dioxane, and *N*-methylpyrrolidone;
Method 10, comprising weighing the compound of formula I into an HPLC bottle, adding a solvent into the HPLC bottle, heating and stirring for equilibration, then filtering, and collecting a supernatant; placing the supernatant into a biological incubator, cooling from 50 °C to 5 °C at 0.05 °C/min and then maintaining the temperature at 5 °C, transferring the clarified solution to -20 °C and maintaining the temperature, collecting a precipitated solid, and transferring the sample without solid precipitation to the room temperature for volatilization,
wherein the solvent is selected from one or more of isopropanol, anisole, isopropyl acetate, tetrahydrofuran, and water;
preferably, the target temperature of the heating is 45-55 °C, preferably 50 °C; and
Method 11, comprising stirring a suspension of the compound of formula I, performing centrifugation, and collecting a solid,
wherein a solvent in the suspension is selected from one or more of *n*-BuOH, toluene, isopropyl ether, methylcyclohexane, cumene, anisole, water, petroleum ether, dicyclohexylamine, 2-methyltetrahydrofuran, n-hexane, 2-butanone, isopropyl acetate, trichloromethane, *m*-xylene, tetrahydrofuran, methyl isobutyl ketone, cyclopentyl methyl ether, and benzyl alcohol;
preferably, the suspension is stirred at 45-55 °C.

13. A preparation method for the free base crystalline form B of the compound of formula I as claimed in claim 3, comprising the following steps:
dissolving free base crystalline form A of the compound of formula I in 1,4-dioxane, and then performing gas-liquid diffusion in an *n*-hexane atmosphere to give the free base crystalline form B of the compound of formula I.

14. A preparation method for the pharmaceutically acceptable salt of the compound of formula I as claimed in claim 11, comprising the following step: mixing the compound of formula I or free base crystalline form A of the compound of formula I with a salt-forming reagent in a proper solvent to give a mixture,
wherein preferably, the salt-forming reagent is the inorganic acid or the organic acid as claimed in claim 4; the free base crystalline form A of the compound of formula I is the free base crystalline form A defined for the crystalline form as claimed in claim 2.

15. A pharmaceutical composition, comprising one or more of the crystalline forms of the compound of formula I or the pharmaceutically acceptable salt thereof as claimed in any one of claims 1-10 and the pharmaceutically acceptable salt of the compound of formula I as claimed in claim 11,
wherein preferably, the pharmaceutically acceptable salt of the compound of formula I is a hydrochloride, a maleate, a *p*-toluenesulfonate, a benzene sulfonate, or a malonate of the compound of formula I;
preferably, the crystalline form of the salt is the crystalline form A of hydrochloride, the crystalline form A of maleate, the crystalline form A of p-toluenesulfonate, the crystalline form A of benzene sulfonate, or the crystalline form A of malonate of the compound of formula I.

16. Use of the crystalline form of the compound of formula I or the pharmaceutically acceptable salt thereof as claimed in any one of claims 1-10, the pharmaceutically acceptable salt of the compound of formula I as claimed in claim 11, or the pharmaceutical composition as claimed in claim 15 for the manufacturing of a therapeutic medicament,
wherein preferably, the medicament is used for the treatment and/or prevention of a P2X3-related disease; preferably, the P2X3-related disease comprises: pain, genitourinary system diseases, or respiratory system diseases;
preferably, the pain comprises: inflammatory pain, surgical pain, visceral pain, dental pain, premenstrual pain, central pain, burn-induced pain, migraine, or cluster headache; preferably, the genitourinary system diseases comprise: urinary incontinence, overactive bladder, dysuria, cystitis, endometriosis, and endometriosis-associated pain; preferably, the respiratory system diseases comprise: cough, idiopathic pulmonary fibrosis, and chronic obstructive pulmonary disease;
preferably, the cough comprises subacute or chronic cough, treatment-resistant cough, idiopathic chronic cough, post-viral cough, iatrogenic cough, and cough associated with respiratory system diseases.

17. A method for treating and/or preventing a P2X3-related disease, comprising administering to a patient a therapeutically effective dose of the crystalline form of the compound of formula I or the pharmaceutically acceptable salt thereof as claimed in any one of claims 1-10, the pharmaceutically acceptable salt of the compound of formula I as claimed in claim 11, or the pharmaceutical composition as claimed in claim 15.

18. A quality detection method for the crystalline form as claimed in any one of claims 1-10, comprising detecting the content of the crystalline form by using high-performance liquid chromatography, wherein mobile phases adopted in the high-performance liquid chromatography comprise a mobile phase A and a mobile phase B;
the mobile phase A is an aqueous solution of formic acid (FA) and acetonitrile (ACN); the mobile phase B is acetonitrile;
preferably, the mobile phase A is an aqueous solution of 0.05%-0.15% formic acid and 2%-7% acetonitrile, illustratively an aqueous solution of 0.1% formic acid and 5% acetonitrile;
preferably, the quality detection method comprises: a purity detection method, a solubility detection method, and a stability detection method;
preferably, the high-performance liquid chromatography adopts gradient elution;
preferably, the flow rate of the mobile phases is 1±0.2 mL/min; the gradient elution is performed for 5-60 min, more preferably 10-30 min;
preferably, in the gradient elution, the mobile phase A and the mobile phase B are in a volume ratio of 1:9 to 9:1.
